# FASCICULE DE BREVET EUROPEEN

(11) **EP 2 430 044 B1**
(45) Date de publication et mention de la délivrance du brevet: **06.03.2019**
(21) Numéro de dépôt: 10730816.5
(22) Date de dépôt: 12.05.2010
(51) Int. Cl.: C07K 14/47, A61K 38/17, A23C 9/127, A23L 33/18, A23L 33/19, A23C 9/123

(54) **PEPTIDES AUGMENTANT LA SECRETION ET/OU L'EXPRESSION D'AU MOINS UNE MUCINE GASTRO-INTESTINALE ET/OU INDUISANT L'AUGMENTATION DE LA POPULATION DE CELLULES A MUCUS OU DE CELLULES DE PANETH.**
PEPTIDE, DIE DIE SEKRETION VON MINDESTENS EINER MAGEN-DARM MUCIN ERHÖHEN UND/ODER INDUZIEREN DIE ERHÖHUNG VON SCHLEIMZELLEN ODER PANETHZELLEN.
PEPTIDES INCREASING THE SECRETION AND/OR EXPRESSION OF AT LEAST ONE GASTROINTINAL MUCINE AND/OR INDUCING THE INCREASE OF MUCUS CELLS OR PANETH CELLS.

(30) Priorité: 13.05.2009 FR 0953165
(43) Date de publication de la demande: 21.03.2012
(73) Titulaire: Institut National de la Recherche Agronomique (INRA), 75338 Paris Cedex 07 (FR)
(72) Inventeur: PLAISANCIÉ, Pascale, F-69003 Lyon (FR); CLAUSTRE, Jean, F-69008 Lyon (FR); ESTIENNE, Monique, F-69100 Villeurbanne (FR); JOURDAN, Gérard, F-69680 Chassieu (FR); LEONIL, Joëlle, F-35000 Rennes (FR); HENRY, Gwénaële, F-35890 Laillé (FR); MOLLE, Daniel, F-35310 Chavagne (FR); MADEC, Marie-Noëlle, F-35000 Rennes (FR); GOGLY, Rachel, F-35310 Chavagne (FR); PAQUET, Armelle, F-38290 La Verpilliere (FR)
(74) Mandataire: Lavoix
(86) Numéro de dépôt international: PCT/FR2010/050926
(87) Numéro de publication internationale: WO 2010/130956

(56) Documents cités:
- WO-A-01/11937
- WO-A-2004/092206
- PERPETUO E A ET AL: "Biochemical and pharmacological aspects of two bradykinin-potentiating peptides obtained from tryptic hydrolysis of casein" JOURNAL OF PROTEIN CHEMISTRY, XX, XX, vol. 22, no. 7-8, 1 novembre 2003 (2003-11-01), pages 601-606, XP002980616 ISSN: 0277-8033
- DATABASE UniProt [Online] 25 juillet 2006 (2006-07-25), "SubName: Full=Beta casein; Flags: Fragment;" XP002602910 extrait de EBI accession no. UNIPROT:Q155X6 Database accession no. Q155X6
- XU R-J: "BIOACTIVE PEPTIDES IN MILK AND THEIR BIOLOGICAL AND HEALTH IMPLICATIONS" FOOD REVIEWS INTERNATIONAL, NEW YORK, NY, US, vol. 14, no. 1, 1 février 1998 (1998-02-01), pages 1-16, XP000913505 cité dans la demande
- TROMPETTE AURELIEN ET AL: "Milk bioactive peptides and beta-casomorphins induce mucus release in rat jejunum." JOURNAL OF NUTRITION, vol. 133, no. 11, novembre 2003 (2003-11), pages 3499-3503, XP002556574 ISSN: 0022-3166 cité dans la demande
- ZOGHBI SANDRA ET AL: "beta-Casomorphin-7 regulates the secretion and expression of gastrointestinal mucins through a mu-opioid pathway" AMERICAN JOURNAL OF PHYSIOLOGY - GASTROINTESTINAL AND LIVER PHYSIOLOGY, vol. 290, no. 6, juin 2006 (2006-06), pages G1105-G1113, XP002556575 ISSN: 0193-1857 cité dans la demande
- CLAUSTRE JEAN ET AL: "Effects of peptides derived from dietary proteins on mucus secretion in rat jejunum" AMERICAN JOURNAL OF PHYSIOLOGY, vol. 283, no. 3 Part 1, septembre 2002 (2002-09), pages G521-G528, XP002556576 ISSN: 0002-9513 cité dans la demande
- SCHIEBER ANDREAS ET AL: "Characterization of oligo- and polypeptides isolated from yoghurt" EUROPEAN FOOD RESEARCH AND TECHNOLOGY, vol. 210, no. 5, 2000, pages 310-313, XP002557485 ISSN: 1438-2377 cité dans la demande
- JINSMAA Y ET AL: "Enzymatic release of neocasomorphin and beta-casomorphin from bovine beta-casein" PEPTIDES, ELSEVIER, AMSTERDAM, vol. 20, no. 8, 1 janvier 1999 (1999-01-01), pages 957-962, XP002980615 ISSN: 0196-9781 cité dans la demande
- PASCALE PLAISANCIE ET AL: "Beta-casein (94-123) - derived peptides differently modulate production of mucins in intestinal goblet cells", JOURNAL OF DAIRY RESE, CAMBRIDGE UNIVERSITY PRESS, CAMBRIDGE, GB, vol. 82, 22 October 2014 (2014-10-22), pages 36-46, XP008182739, ISSN: 0022-0299

### SEQUENCE LISTING

<110> INRA
<120> Composés augmentant la sécrétion et/ou l'expression d'au moins une mucine gastro-intestinale et/ou induisant l'augmentation de la population de cellules à mucus ou de cellules de Paneth
<130> BEX10P0366
<150> FR0953165
   <151> 2009-05-13
<160> 2
<170> PatentIn version 3.4
<210> 1
   <211> 30
   <212> PRT
   <213> Artificial sequence
<220>
   <223> peptide
<400> 1
   Gly Val Ser Lys Val Lys Glu Ala Met Ala Pro Lys Gln Lys Glu Met
   1               5                   10                  15
   Pro Phe Pro Lys Tyr Pro Val Glu Pro Phe Thr Glu Ser Gln
               20                  25                  30
<210> 2
   <211> 7
   <212> PRT
   <213> Artificial sequence
<220>
   <223> peptide
<400> 2
   Tyr Pro Phe Pro Gly Pro Ile
   1               5

## Description

La présente invention a pour objet des composés permettant d'induire une augmentation de l'expression et/ou de la synthèse et/ou de la sécrétion d'au moins une mucine gastro-intestinale. La présente invention a également pour objet des composés permettant d'induire au moins une molécule de défense intestinale exprimée par les cellules de Paneth telle que le lysozyme ou une alpha-défensine. La présente invention a, enfin, pour objet des composés permettant d'induire une augmentation de la population des cellules à mucus et/ou des cellules de Paneth.

L'épithélium intestinal est organisé en cryptes et villosités dont les cellules sont renouvelées de façon rapide et continue. Ce renouvellement s'effectue à partir de cellules souches multipotentes qui sont localisées dans le bas des cryptes. Ces cellules peuvent se diviser soit de façon symétrique (10% des cas) en générant deux autres cellules souches qui resteront dans leur niche, soit de façon asymétrique (90% des cas) en donnant une nouvelle cellule souche et une cellule fille progénitrice destinée à se différencier. Ces cellules progénitrices évolueront ensuite vers deux grandes familles de cellules spécialisées capables d'assurer les fonctions intestinales de transport, de sécrétion (exocrine et endocrine) et de défense de l'organisme: les cellules absorptives (entérocytes) et les cellules du lignage sécrétoire qui comprend les cellules caliciformes (ou cellules à mucus), les cellules de Paneth et les cellules entéro-endocrines.

Le mucus gastro-intestinal est connu pour son rôle dans la protection de la surface de l'épithélium du tractus gastro-intestinal. Cette protection permet de lutter contre les agressions telles que l'acide, les enzymes digestives, les toxines, ou l'alcool, et constitue également une barrière naturelle contre certains types de pathogènes (Deplancke et al. (2001) Am J Clin Nutr 73(6) :1131S-1141S). Des défauts dans l'établissement de ce mucus peuvent donc induire une altération de la barrière muqueuse et sont à l'origine de nombreuses pathologies dues en particulier à une inflammation des muqueuses gastriques et intestinales (Gibson et al. (2005) Gut 54:900-903, Einerhand et al. (2002) Eur J Gastroenterol Hepatol 14(7):757-65).

Les principaux composants du mucus sont des glycoprotéines de la famille des mucines. Il s'agit de polymères de grandes tailles, composés de monomères de glycoprotéines hautement glycosylées. Quatre membres de cette famille sont connus pour être impliqués dans la formation du gel de mucus : MUC2, MUC5AC, MUC5B et MUC6 (Desseyn et al. (2000) Mol Biol Evol 17(8) :1175-84). Un second groupe de mucines associées à la membrane, auquel appartiennent MUC1, MUC3 et MUC4, joue également un rôle dans la protection de l'épithélium.

MUC2 est la principale mucine présente dans le mucus intestinal. Il a été montré récemment que l'invalidation du gène *muc2* chez la souris se traduit par l'apparition de colites et de tumeurs intestinales (Velcich et al. (2002) Science 295(5560) :1726-9). Des modifications de l'expression de cette mucine ont également été observées lors de maladies inflammatoires intestinales.

La mucine MUC2 est produite et secrétée par des cellules épithéliales spécialisées : les cellules à mucus ou cellules caliciformes.

Les cellules de Paneth quant à elles expriment la mucine MUC4 (Rong et al. (2005) J cell Physiol 202(1) :275-84) et se caractérisent par la présence, à leur pôle apical, de micro-granules contenant notamment du lysozyme, de la phospholipase A2 et des alpha-défensines (peptides naturels à vocation d'antibiotiques). Les cellules de Paneth contribuent ainsi à l'immunité innée et représentent un système de défense important permettant de maintenir un environnement stérile dans la lumière du bas des cryptes. De nombreuses données bibliographiques soulignent de fait le rôle de ces cellules dans la défense intestinale. Un défaut en lysozyme dans les cellules de Paneth a été fortement associé à l'entérocolite nécrosante du nouveau-né (Coutinho et al. (1998) J Clin Pathol 51(7) :512-4). Récemment, il a été montré que la maturation des cellules de Paneth est suivie de la mort des bactéries atteignant le jéjunum et qu'une déplétion des cellules de Paneth prédispose aux infections à *Shigella* (Fernandez et al. (2008) J Immunol 180(7) :4924-30). L'intestin de souris déplété en cellules de Paneth se caractérise également par une plus grande pénétration de la barrière de mucus par les bactéries commensales (Vaishnava et al. (2008) Proc Natl Acad Sci USA 105(52) :20858-63). Chez l'homme, une diminution de la production des- alpha-défensines HD-5 et HD-6 par les cellules de Paneth a été décrite dans les formes iléales de la maladie de Crohn. Ce déficit se traduit par une diminution de la capacité de la muqueuse à éliminer différentes souches bactériennes *d'Escherichia coli,* mais aussi de *Staphylococus aureus,* ou de bactéries anaérobies. Enfin, il est important de noter que les cellules de Paneth n'interviennent pas uniquement vis-à-vis des bactéries potentiellement pathogènes. Leur activité anti-microbienne régule également la composition du microbiote commensal. Salzman et al (2010) Nat Immunol 11(1) :76-83) ont en effet démontré que des souris Mmp7-/-, qui ne produisent pas d'α-défensines fonctionnelles, ont un ratio Firmicutes/Bacteroidetes significativement plus haut que les souris sauvages. De façon intéressante, ce type de ratio est également observé chez les sujets obèses ainsi que dans différents modèles animaux d'obésité (régime hyperlipidique, souris ob/ob, ..).

Peu d'éléments sont connus à l'heure actuelle sur la régulation de la sécrétion et de l'expression des mucines gastro-intestinales ou des molécules produites par les cellules de Paneth par les facteurs nutritionnels. Certains travaux ont permis de caractériser l'effet sécrétagogue de fibres alimentaires et d'acides gras à chaîne courte sur les cellules à mucus (Lundin et al. (1993) Scand J Gastroenterol 28(1) :15-22, Sharma et al. (1995) Dig Dis Sci 40(12) :2532-9, Sharma et al. (1995) Lab Invest 73(4) :558-64, Barcelo et al. (2000) Gut 46(2) :218-24, Satchithanandam et al. (1990) J Nutr 120(10) :1179-84, Burger-van Paasen et al. (2009) Biochem J. Feb 2009 DOI BJ20082222, Hedemann et al. (2009) Brit J Nutr, 13 : 1-9).

Plus récemment, des travaux ont porté sur l'effet des β-casomorphines sur la sécrétion des mucines. Les β-casomorphines sont une famille de peptides opioïdes exogènes (exorphines) issus de l'hydrolyse d'une protéine de lait, la caséine β (ou β-caséine). Ces peptides sont libérés durant les différentes étapes de la digestion des protéines du lait dans l'estomac et l'intestin grêle et seraient capables de traverser les monocouches de cellules intestinales (Meisel et al. (2000) BR J Nutr 84(Suppl 1) :S27-31, Sienkiewicz-Szlapka et al. (2009) Int Dairy J 19 :252-257). Ils sont également libérés lors de la fermentation des produits laitiers. Des précurseurs des β-casomorphines ont ainsi été identifiés dans les produits fermentés et notamment dans le yaourt et le fromage (Schieber et al. (2000) Eur Food Res Technol 210 :310-13, Sienkiewicz-Szlapka et al. (2009) Int Dairy J 19 :258-263).

Le principal représentant de cette famille, la β-casomorphine-7 correspond au fragment [60-66] de la β-caséine bovine. Il a été montré que la β-casomorphine-7 est un puissant sécrétagogue du mucus *ex vivo* dans le jéjunum de rat (Trompette et al. (2003) J Nutr 133(11) :3499-503, Claustre et al. (2002) Am J Physiol Gastrointest Liver Physiol 283(3) :G521-8) et qu'elle induit *in vitro* l'augmentation de l'expression de la principale mucine sécrétée MUC2 dans la lignée colique mucipare de rat (DHE) (Zoghbi et al. (2006) Am J Physiol Gastrointest Liver Physiol 290(6) :G1105-13).

En 1999, un nouveau peptide opiacé a été identifié dans la séquence de la caséine β bovine, la séquence [114-119], appelé néocasomorphine-6 (Jinsmaa et al. (1999) Peptides 20 :957-962).

### Description de l'invention

La présente invention découle de la mise en évidence inattendue, par les inventeurs, de ce qu'un nouveau peptide issu de la caséine β bovine, correspondant aux résidus 94-123 (SEQ ID NO : 1) de cette protéine, est un stimulant de l'expression et de la sécrétion de mucines gastro-intestinales, est un stimulant de l'expression du lysozyme produit par les cellules de Paneth, et induit une augmentation de la population des cellules à mucus et des cellules de Paneth.

Ainsi la présente invention est définit par les revendications.

Ainsi, la présente demande décrit un polypeptide comprenant ou constitué de :
- SEQ ID NO : 1, ou
- une séquence présentant au moins 80% d'identité avec SEQ ID NO : 1, ou
- une séquence d'au moins quatre acides aminés consécutifs comprise dans SEQ ID NO : 1,
qui présente au moins un effet choisi parmi :
- l'induction de l'expression et/ou de la sécrétion d'au moins une mucine gastro-intestinale ; et
- l'induction de l'expression et/ou de la sécrétion d'au moins une molécule de défense intestinale exprimée par les cellules de Paneth ; et
- l'induction de l'augmentation de la population des cellules à mucus et/ou de la population des cellules de Paneth,
et sous réserve que le polypeptide ne comprenne pas la séquence SEQ ID NO : 2. De préférence le polypeptide tel que défini ci-dessus est constitué de SEQ ID NO : 1.

SEQ ID NO : 2 correspond à la séquence constituée des acides aminés 60 à 66 de la caséine β bovine.

On entend par "pourcentage d'identité" entre deux séquences polypeptidiques le pourcentage d'acides aminés identiques entre les deux séquences à comparer. Les comparaisons entre deux séquences polypeptidiques sont généralement réalisées après avoir aligné ces deux séquences de manière optimale sur la totalité de leur longueur. Cet alignement peut être réalisé manuellement ou à l'aide d'un programme informatique. A titre d'exemple de programme informatique, on peut citer le programme EMBOSS-Needle (alignement global Needleman-Wunsch) avec l'aide de la matrice BLOSUM62/Open Gap 10.0 et Extension Penalty de 0,5 (Needleman et al. (1970) J. Mol. Biol. 48:443-453; Kruskal (1983) An overview of sequence comparison In D. Sankoff and J.B. Kruskal, (ed)). Comme on l'entend ici, le nombre de positions pour lesquelles les acides aminés sont identiques est ensuite divisé par le nombre total d'acides aminés de la plus grande des deux séquences comparées et le pourcentage d'identité est obtenu en multipliant le résultat obtenu par 100. Un polypeptide ayant, par exemple, une identité d'au moins 90% avec le polypeptide de SEQ ID NO : 1 est un polypeptide comportant, au plus, 3 acides aminés modifiés sur 30 acides aminés, par rapport à ladite séquence. En d'autres termes jusqu'à 10% des acides aminés dans la séquence SEQ ID NO : 1 peuvent être supprimés ou substitués par un autre acide aminé ou la séquence peut comporter jusqu'à 10% d'acides aminés en plus ou en moins par rapport au nombre total d'acides aminés de la séquence SEQ ID NO : 1.

De préférence, la séquence d'au moins quatre acides aminés comprend au moins 4, 5, 10, 20 ou 25 acides aminés consécutifs compris dans SEQ ID NO : 1, préférentiellement 20 acides aminés.

Comme on l'entend ici la séquence homologue peut comprendre à la fois les variants naturels de la séquence SEQ ID NO : 1 ainsi que des mutants artificiels.

Par ailleurs, le polypeptide défini ci-dessus peut être modifié, par exemple par le remplacement d'au moins un acide aminé L par un acide aminé D ou par une modification de la nature chimique d'au moins un acide aminé. Ces modifications, ainsi que les mutations artificielles, sont notamment utiles pour augmenter la stabilité du peptide dans l'environnement gastrique et intestinal, en particulier pour augmenter la stabilité des peptides vis-à-vis des protéases et peptidases digestives telles que la trypsine, la chymotrypsine et la pepsine.

Les inventeurs ont trouvé que le peptide selon l'invention est capable d'induire *in vitro* l'expression et/ou la sécrétion de mucines gastro-intestinales.

Par ailleurs, il a été démontré que cet effet d'induction existe également *in vivo* et peut être relié, en particulier, à une augmentation du niveau des transcrits codant les mucines et à une augmentation du nombre de cellules sécrétrices de mucines, en particulier du nombre de cellules à mucus.

L'expression et/ou la sécrétion de mucines gastro-intestinales peut être déterminée de manière aisée par des méthodes bien connues de l'homme du métier comme cela est notamment décrit par El Homsi et al. ((2007) Am J Physiol Gastrointest Liver Physiol 293(1):G365-73), Radhakrishnan et al. ((2007) Biochim Biophys Acta 1770(6):884-9) et Lu et al. ((2005) Am J Physiol Lung Cell Mol Physiol 288(1):L52-60)) et en particulier comme cela est décrit dans l'Exemple 2 suivant.

A titre d'exemple, l'expression d'au moins une mucine gastro-intestinale peut être notamment déterminée *in vitro* et *in vivo* en quantifiant l'ARNm d'au moins une mucine ou la quantité d'au moins une protéine de mucine produite par des cellules intestinales. Une mesure de l'expression d'une mucine par estimation de la quantité d'ARNm codant la mucine dans les cellules à mucus peut notamment être réalisée comme décrit *in vitro* dans l'Exemple 2 et *in vivo* dans l'Exemple 3. Une mesure de l'expression d'une mucine produite par les cellules à mucus par immuno-histochimie sur des coupes d'intestin inclus en paraffine à l'aide d'un anticorps dirigé contre la mucine peut également être réalisée comme décrit dans l'Exemple 3 ou encore par ELISA, ELLA (Enzyme Linked Lectin Assay) et Western blot.

A titre d'exemple, la sécrétion d'au moins une mucine gastro-intestinale peut être déterminée *in vitro* en mesurant la quantité de mucines présentes dans le milieu de culture des cellules mucosécrétantes. Cette mesure peut être réalisée par des méthodes bien connues de l'homme du métier, par exemple, par western blot, par technique ELISA ou ELLA comme notamment décrit dans l'Exemple 2 à l'aide d'une lectine dirigée contre les chaines oligosaccharidiques des mucines.

Les inventeurs ont également montré que le peptide selon l'invention est capable d'induire l'expression et/ou la sécrétion d'au moins une molécule de défense intestinale exprimée par les cellules de Paneth.

Une molécule de défense intestinale exprimée par les cellules de Paneth peut être, par exemple, le lysozyme ou une alpha-défensine en particulier les alpha-défensines HD5 et HD6, ou la phospholipase A2 (sPLA2) ou l'angiogénine 4 (Ang4) ou le secretory leukocyte protease inhibitor (SLPI) ou l'hepatocarcinoma-intestine-pancreas/Pancreatic associated protein (HIP/HAP) ou RegIIIγ (regenerating gene III), préférentiellement le lysozyme.

L'expression d'au moins une molécule de défense intestinale peut être, par exemple, déterminée *in vitro* et *in vivo* en mesurant la quantité d'ARNm codant au moins une molécule de défense intestinale ou la quantité d'au moins une protéine de défense intestinale exprimée par les cellules de Paneth. Une mesure de l'expression du lysozyme peut par exemple être réalisée par estimation de la quantité d'ARNm codant le lysozyme dans les cellules de Paneth notamment comme décrit dans l'Exemple 3. Une mesure de l'expression du lysozyme produit par les cellules de Paneth peut également être réalisée par immuno-histochimie sur des coupes d'intestin inclus en paraffine à l'aide d'un anticorps dirigé contre le lysozyme comme notamment décrit dans l'Exemple 3.

A titre d'exemple, la sécrétion d'au moins une molécule de défense intestinale peut être notamment déterminée *in vivo* en mesurant la quantité de molécules de défense intestinale présentes dans le contenu de la lumière intestinale. Cette mesure peut être réalisée par des méthodes bien connues de l'homme du métier, par exemple, par western blot, ou par technique ELISA à l'aide d'un anticorps dirigé contre la molécule de défense à doser

Les inventeurs ont également montré que le peptide selon l'invention est capable d'induire l'augmentation de la population des cellules à mucus et/ou des cellules de Paneth.

L'augmentation de la population des cellules à mucus et/ou des cellules de Paneth peut, par exemple, être déterminée *in vitro* ou *in vivo* par la mesure du nombre de cellules à mucus et/ou du nombre de cellules de Paneth. Des méthodes de mesure du nombre de cellules à mucus et/ ou de cellules de Paneth sont bien connues de l'homme du métier. Par exemple, le nombre de cellules peut être déterminé *in vivo* ou *in vitro* par comptage par exemple, sur des coupes d'intestin inclus en paraffine et immunomarquées à l'aide d'anticorps spécifiques, notamment comme décrit dans l'Exemple 3.

Les cellules mucosécrétantes utilisées *in vitro* sont de préférence des cellules mucosécrétantes intestinales telles que des cellules de la lignée cellulaire humaine HT29-MTX ou HT29-CI16E.

L'expression déterminer l'expression et/ou la sécrétion « d'au moins une » mucine ou l'expression et/ou la sécrétion « d'au moins » une molécule de défense intestinale indique que l'expression d'une, de deux, de trois mucines ou plus ou d'une, deux ou trois molécules de défense intestinale ou plus peut être déterminée. De préférence, la ou les mucines dont l'expression et/ou la sécrétion est déterminée sont sélectionnées parmi les mucines intestinales MUC2, MUC3 et MUC4 ou gastro-intestinales MUC5AC et MUC6. De préférence, il s'agit de la mucine MUC2 et/ou MUC4. De préférence la ou les molécules de défense intestinale dont l'expression et/ou la sécrétion est déterminée sont sélectionnées parmi le lysozyme et les alpha-défensines. De préférence il s'agit du lysozyme.

De préférence, l'effet d'induction de l'expression et/ou de la sécrétion d'au moins une mucine gastro-intestinale ou d'au moins une molécule de défense intestinale correspond à une augmentation de l'expression et/ou de la sécrétion d'au moins une de ces mucines ou d'au moins une de ces molécule de défense intestinale d'au moins 50%, 60%, 70%, 80%, 90% ou 100% par rapport à l'expression et/ou la sécrétion obtenue en absence de peptide. De préférence également, l'effet d'induction de l'expression et/ou de la sécrétion d'au moins une mucine gastro-intestinale ou d'au moins une molécule de défense intestinale correspond à une augmentation de l'expression et/ou de la sécrétion d'au moins une de ces mucines ou d'au moins une de ces molécules de défense intestinale d'au moins 50%, 60%, 70%, 80%, 90% ou 100% par rapport à l'expression et/ou la sécrétion obtenue avec un polypeptide constitué de SEQ ID NO : 1 mesurée dans les mêmes conditions.

Dans un mode de réalisation particulier, la demande décrit le polypeptide tel que défini ci-dessus pour son utilisation comme médicament, de préférence dans le traitement de patients présentant un défaut d'expression et/ou de sécrétion de mucines gastro-intestinales et/ou une altération de la barrière muqueuse gastro-intestinale.

Les pathologies liées à un défaut d'expression et/ou de sécrétion de mucines gastro-intestinales et/ou à une altération de la barrière muqueuse gastro-intestinale comprennent toutes les pathologies directement ou indirectement liées à une absence ou à une insuffisance de l'expression et/ou de la sécrétion de mucines. De telles pathologies peuvent être dues à une déplétion, un dysfonctionnement ou une insuffisance des cellules à mucus. De telles pathologies sont bien connues de l'homme du métier et sont notamment décrites dans Turner et al. (2003) Cancer Chemother Biol Response Modif 21:259-74), Utsunomiya et al. (1998) Clin Cancer Res 4:2605-14 et Nakamori et al. (1994) Gastroenterol 106:353-361.

De préférence, les pathologies liées à un défaut d'expression et/ou de sécrétion de mucines gastro-intestinales et/ou une altération de la barrière muqueuse gastro-intestinale sont sélectionnées dans le groupe constitué de l'inflammation intestinale notamment chronique, d'une infection intestinale, d'ulcère gastrique ou duodénal, notamment dus à une infection à *Helicobacter pylori,* de diarrhée, de constipation, de cancers intestinaux et coliques et des mucites induites par la radiothérapie ou la chimiothérapie. Dans un autre mode de réalisation, la demande décrit le polypeptide tel que défini ci-dessus pour son utilisation comme médicament dans la prévention ou le traitement de patients atteints de pathologies pouvant être prévenues ou traitées par une action sur les cellules de Paneth, en particulier dans la prévention ou le traitement de patients présentant une déplétion et/ou un dysfonctionnement des cellules de Paneth. Préférentiellement, une déplétion ou un dysfonctionnement des cellules de Paneth est du à une diminution de cette population cellulaire ou à un défaut de maturation ou un défaut en lysozyme ou en une autre molécule de défense, notamment une alpha-défensine, des cellules de Paneth.

Encore préférentiellement, la demande décrit le polypeptide tel que défini ci-dessus pour son utilisation comme médicament dans la prévention ou le traitement d'une pathologie choisie parmi une pathologie liée à un défaut d'expression et/ou de sécrétion d'une molécule de défense intestinale exprimée par les cellules de Paneth, une infection due à des bactéries, virus, levures ou protozoaires, l'entérocolite nécrosante du nourrisson, les diarrhées, la maladie de Crohn et autres inflammations intestinales et coliques et le traitement de patients présentant une altération du microbiote intestinal, comme observée par exemple lors d'obésité ou de maladies métaboliques.

Les infections bactériennes sont préférentiellement choisies parmi les infections dues à *Shigella, Salmonella, Staphylococcus aureus ou à Listeria.*

Les traitements selon l'invention peuvent être administrés à la suite de l'apparition de ces désordres ou de façon préventive. Le traitement selon l'invention peut être administré aux animaux, en particulier aux mammifères, et notamment l'homme.

La présente demande décrit également une composition pharmaceutique comprenant un polypeptide tel que défini ci-dessus à titre de substance active, en association avec des excipients ou véhicules pharmaceutiquement acceptables et, éventuellement, à des matrices à libération soutenue comme, par exemple, des polymères biodégradables pour former des compositions thérapeutiques.

Le terme "pharmaceutiquement acceptable" se rapporte à des molécules et compositions qui n'induisent pas une réaction adverse, allergique ou non désirée lorsqu'elles sont administrées à un mammifère, en particulier à un humain. Un véhicule pharmaceutiquement acceptable ou excipient peut être un solide ou un semi-solide, un liquide, un diluant, un matériel encapsulé ou n'importe quelle autre formulation accessoire.

La forme de la composition pharmaceutique, le mode d'administration, le dosage et la posologie dépendent bien sûr, entre autres, de la maladie à traiter, de sa sévérité, de l'âge, du poids et du sexe du patient.

La composition pharmaceutique selon l'invention est préférentiellement formulée de façon à pouvoir être administrée par voie orale ou entérale.

La composition pharmaceutique selon l'invention peut être une composition alimentaire. La présente demande décrit donc également l'utilisation d'un polypeptide tel que défini ci-dessus, pour la préparation d'une composition alimentaire, de préférence une composition alimentaire lactée. Ce type de composition alimentaire peut notamment être qualifié d'alicament ou de nutraceutique.

Les compositions alimentaires lactées selon l'invention peuvent être, de façon non limitative, des yaourts, des fromages ou des boissons lactées. Dans un mode de réalisation préféré, les fromages sont exclus de la présente invention.

La présente demande décrit également une composition alimentaire lactée ou non lactée, préférentiellement lactée, comprenant un polypeptide tel que défini ci-dessus préférentiellement à des concentrations comprises entre 0.001 et 100 µM, par exemple, dans un mode de réalisation particulier la concentration peut être d'au moins 7 µM, encore préférentiellement supérieure à 10 µM et de préférence inférieure à 15 µM, encore de préférence inférieure à 20 µM. Dans un mode de réalisation préféré, le polypeptide est à une concentration comprise entre 0.001 et 10µM, 0.001 et 1µM, 0.005 et 0.5µM, 0.005 et 0.05µM, 0.01 et 0.1µM, 0.01 et 1µM, encore préférentiellement le polypeptide est à une concentration égale à environ 0.01µM.

Les polypeptides selon l'invention peuvent êtres obtenus par de nombreuses méthodes bien connues de l'homme du métier.

Ainsi, les polypeptides peuvent être synthétisés au préalable, par exemple par voie chimique ou par production par une bactérie recombinante capable de synthétiser un tel polypeptide. De façon préférentielle, de telles bactéries sont capables de synthétiser et de secréter les peptides d'intérêt. Les peptides obtenus sont alors extraits du surnageant de la culture bactérienne.

Alternativement ou de manière complémentaire, la présence du polypeptide, tel que défini ci-dessus dans les compositions alimentaires, peut être due à la dégradation de la caséine β bovine par l'action de bactéries fermentatives.

La présente demande décrit donc également un procédé de préparation d'un produit lacté fermenté par exemple, par fermentation par au moins une souche de *Lactobacillus delbrueckii* ssp. *bulgaricus* et/ou au moins une souche de *Streptococcus salivarus* spp. *thermophilus* comprenant une étape de détermination de la concentration d'un polypeptide tel que défini ci-dessus. De préférence, le procédé est tel qu'on laisse agir la souche de *Lactobacillus delbrueckii* ssp. *bulgaricus* et/ou la souche de *Streptococcus salivarus* spp. *thermophilus* jusqu'à ce qu'une valeur prédéterminée de la concentration du polypeptide soit atteinte.

Les méthodes de fermentation des produits lactés, tels que yaourts et fromages, sont bien connues de l'homme du métier. Les ferments lactiques pouvant être couramment utilisés reposent notamment sur l'association d'une souche de *Lactobacillus delbrueckii* ssp. *bulgaricus* et/ou d'une souche de *Streptococcus salivarus* spp. *thermophilus.*

### Description des figures

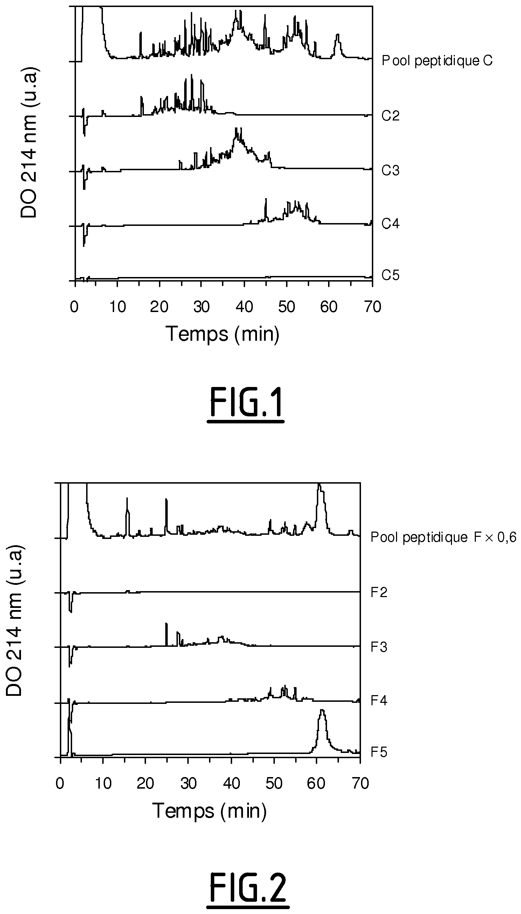

**FIG. 1** : Profils obtenus en RP-HPLC pour le pool peptidique total issus du yaourt du commerce (noté pool peptidique C) et les différentes fractions obtenues à partir de ce pool peptidique (notées C2 à C5). La fraction C1 était retrouvée en trop faible quantité pour être analysée.
**FIG. 2** : Profils obtenus en RP-HPLC pour le pool peptidique total issus du yaourt préparé au laboratoire (noté pool peptidique F) et les différentes fractions obtenues à partir de ce pool peptidique (notées F2 à F5). La fraction F1 était retrouvée en trop faible quantité pour être analysée.

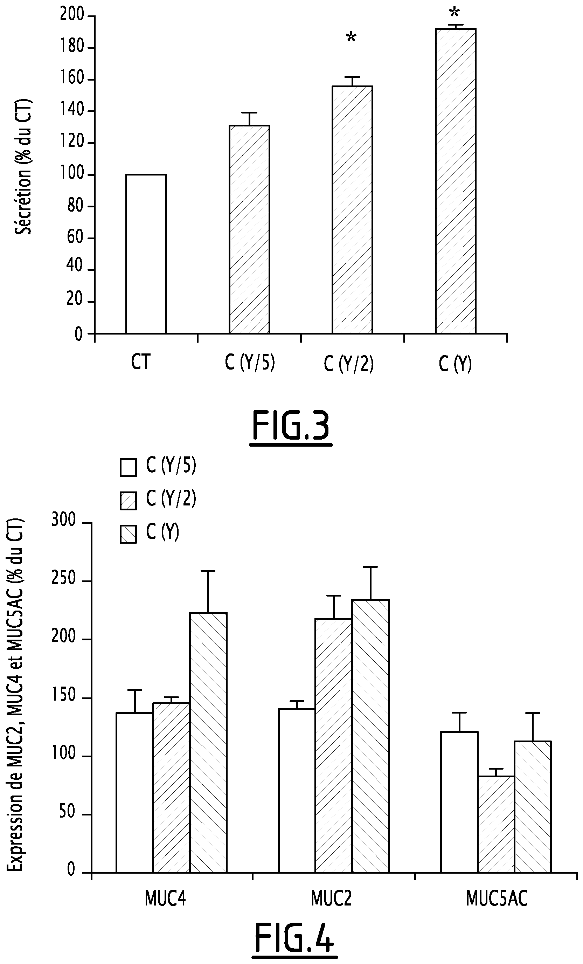

**FIG. 3** **:** Effet du pool peptidique total du yaourt du commerce testé aux concentrations Y/5, Y/2 et Y (horizontalement) sur la sécrétion de mucines par la lignée HT29-MTX après 4 h de stimulation, exprimé en pourcentage de la sécrétion de mucines après 4h d'incubation en absence de pool peptidique: CT (verticalement). Le symbole étoile (*) correspond à une probabilité p<0,05 par rapport aux contrôles. Y correspond à la MAT des peptides dans le yaourt initial.
**FIG. 4** **:** Effet du pool peptidique total du yaourt du commerce testé aux concentrations Y, Y/2 et Y/5 (horizontalement), sur l'expression des gènes *muc2, muc4* et *muc5AC* par la lignée HT29-MTX après 4 h de stimulation, exprimé en pourcentage de l'expression de mucines après 4h d'incubation en absence de pool peptidique: CT (verticalement).

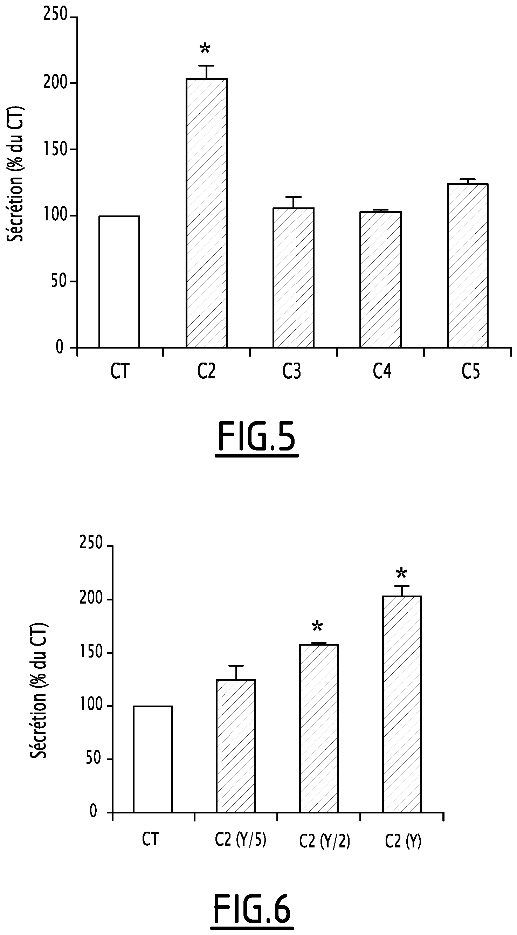

**FIG. 5** : Effet des différentes fractions C2 à C5, à la concentration Y, issues du yaourt du commerce (horizontalement) sur la sécrétion de mucines par la lignée HT29-MTX après 4 h de stimulation, exprimé en pourcentage de la sécrétion de mucines après 4h d'incubation en absence de fraction: CT (verticalement). Le symbole étoile (*) correspond à une probabilité p<0,05 par rapport aux contrôles.
**FIG. 6** : Effet de différentes concentrations (Y/5, Y/2, Y) de la fraction C2 issue de yaourt du commerce (horizontalement) sur la sécrétion de mucines par la lignée HT29-MTX, après 4 h de stimulation, exprimé en pourcentage de la sécrétion de mucines après 4h d'incubation en absence de fraction: CT (verticalement). Le symbole étoile (*) correspond à une probabilité p<0,05 par rapport aux contrôles.

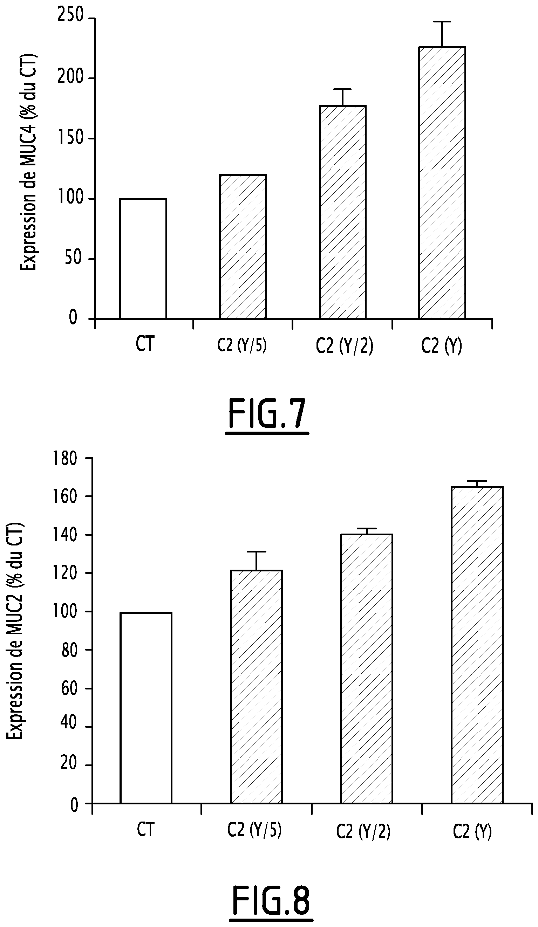

**FIG. 7** : Effet de différentes concentrations (Y/5, Y/2, Y) de la fraction C2 issue du yaourt du commerce (horizontalement) sur l'expression du gène *muc4* par la lignée HT29-MTX après 4 h de stimulation, exprimé en pourcentage de l'expression de mucines après 4h d'incubation en absence de fraction: CT (verticalement).
**FIG. 8** : Effet de différentes concentrations (Y/5, Y/2, Y) de la fraction C2 issue du yaourt du commerce (horizontalement) sur l'expression du gène *muc2* par la lignée HT29-MTX après 4 h de stimulation, exprimé en pourcentage de l'expression de mucines après 4h d'incubation en absence de fraction: CT (verticalement).

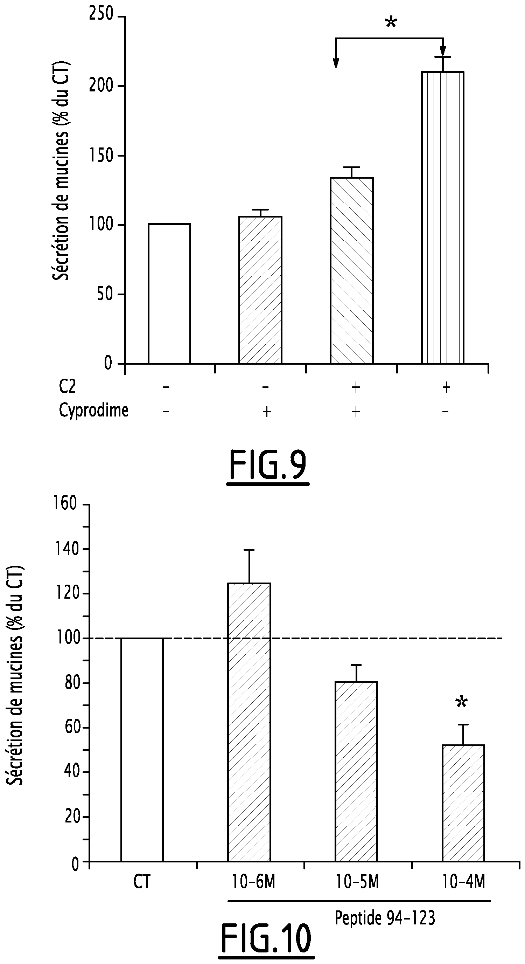

**FIG. 9** **:** Effet sur la sécrétion de mucines par la lignée HT29-MTX après pré-incubation de 10 minutes en présence ou non de cyprodime, suivi de 4 h de stimulation avec de la fraction C2 (à la concentration Y) en présence ou non du cyprodime exprimé en pourcentage de la sécrétion de mucines par la lignée HT29-MTX en absence de cyprodime et de fraction C2. * correspond à une probabilité p<0,05 par rapport aux contrôles.
**FIG. 10** : Effet de différentes concentrations (10⁻⁶M, 10⁻⁵M, 10⁻⁴M) du peptide [94-123] (horizontalement) sur la sécrétion de mucines par la lignée HT29-MTX, après 4 h d'incubation, exprimé en pourcentage de la sécrétion de mucines après 4h d'incubation en absence de peptide: CT (verticalement). Le symbole étoile (*) correspond à une probabilité p<0,05 par rapport aux contrôles.

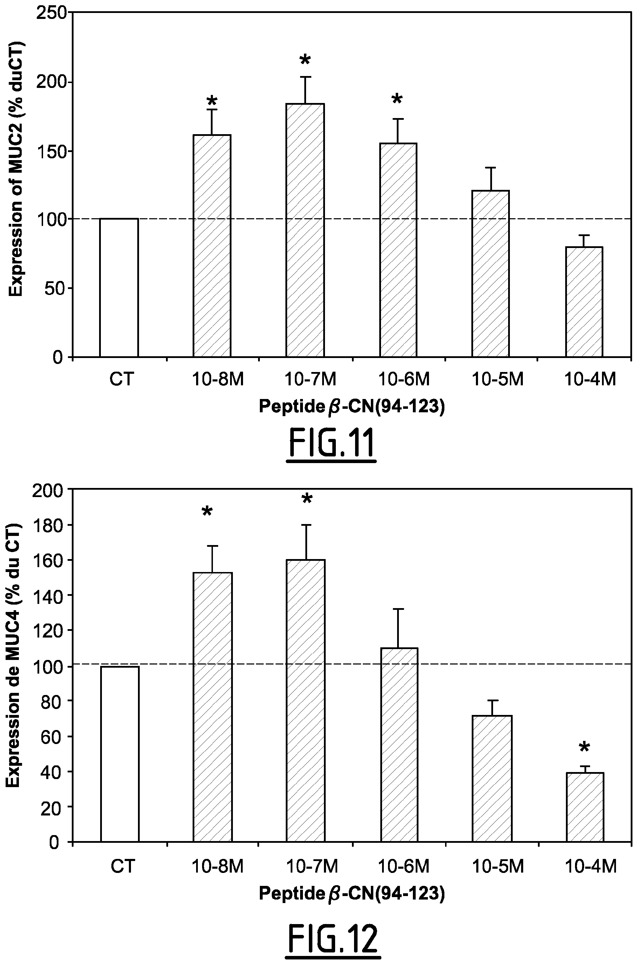

**FIG. 11** : Effet de différentes concentrations (10⁻⁸M à 10⁻⁴M) du peptide [94-123] (β-CN(94-123))(horizontalement) sur l'expression du gène *muc2* par la lignée HT29-MTX après 4 h d'incubation, exprimé en pourcentage de l'expression du gène *muc2* des CT (4h d'incubation sans peptide). Données obtenues par qRT-PCR. Le symbole étoile (*) correspond à une probabilité p<0,05 par rapport aux contrôles
**FIG. 12** : Effet de différentes concentrations (10⁻⁸M à 10⁻⁴M) du peptide [94-123] (β-CN(94-123)) (horizontalement) sur l'expression du gène *muc4* par la lignée HT29-MTX après 4 h d'incubation, exprimé en pourcentage de l'expression du gène *muc4* des CT (4h d'incubation sans peptide). Données obtenues par qRT-PCR. Le symbole étoile (*) correspond à une probabilité p<0,05 par rapport aux contrôles

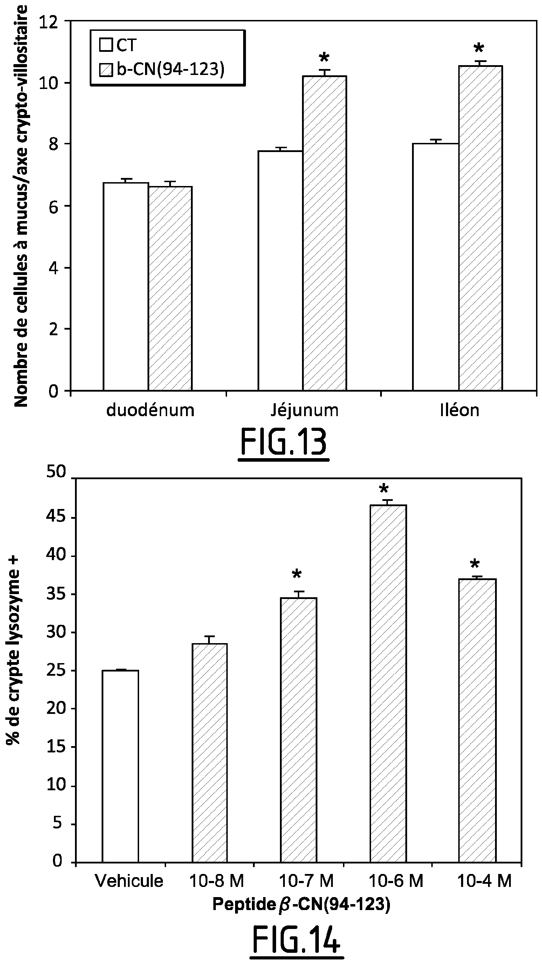

**FIG. 13** **:** Effet de l'administration par sonde gastrique (J10-J18) du peptide [94-123] (β-CN(94-123)) à la concentration de 10⁻⁴M sur le nombre de cellules à mucus par axe crypto-villositaire dans le duodénum, le jéjunum et l'iléon de rat. * p<0.05 vs véhicule. Les données ont été analysées par une Anova à un facteur suivie du test de Bonferroni.
**FIG. 14** **:** Effet de l'administration par sonde gastrique (J10-J18) du peptide [94-123] (β-CN(94-123)) sur les cellules de Paneth du duodénum de rat. * p<0.05 vs véhicule. Les données ont été analysées par une Anova à un facteur suivie du test de Bonferroni.

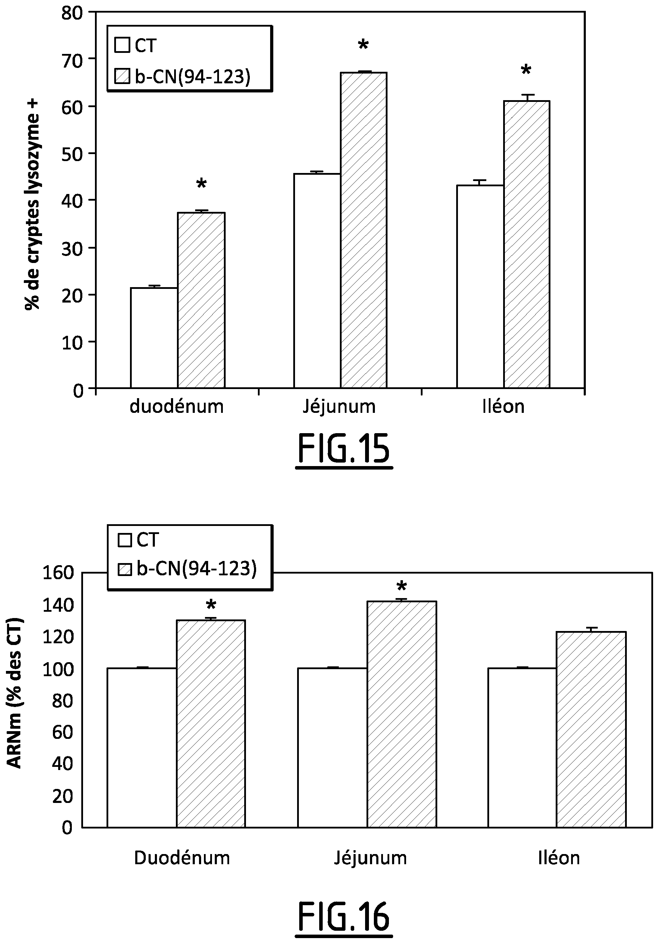

**FIG. 15** **:** Effet de l'administration par sonde gastrique (J10-J18) du peptide [94-123] (β-CN(94-123)) à la concentration de 10⁻⁴M sur les cellules de Paneth du duodénum, du jéjunum et de l'iléon de rat. * p<0.05 vs véhicule. Les données ont été analysées par une Anova à un facteur suivie du test de Bonferroni.
FIG. 16 : Effet de l'administration par sonde gastrique (J10-J18) du peptide [94-123] (β-CN(94-123)) à la concentration de 10⁻⁴M sur l'expression du lysozyme dans le duodénum, le jéjunum et l'iléon de rat (qRT-PCR). * p<0.05 vs véhicule. Les données ont été analysées par une Anova à un facteur suivie du test de Bonferroni.

### EXEMPLES

### EXEMPLE 1 : La protéolyse des caséines par les bactéries lactiques du yaourt entraîne la formation de précurseurs de peptides bioactifs à activité opiacée.

### MATERIEL ET METHODE

### Culture de ferments lactiques

Les ferments lactiques utilisés industriellement pour la production de yaourt reposent toujours sur l'association en culture mixte d'une souche de *Lactobacillus delbrueckii* spp. *bulgaricus* avec une souche de *Streptococcus salivarius spp.* thermophilus, issues de collections à la disposition des fabricants. Les souches de ferments lactiques (Syndifrais) conservées à -18°C étaient revivifiées et repiquées à plusieurs reprises. La souche de *Lactobacillus delbrueckii spp. bulgaricus* était cultivée à 43°C en anaérobie sur milieu MRS (De Man, Rogosa, Sharpe) liquide pH 5,4 tandis que la souche de *Streptococcus salivarius* spp. *thermophilus* était cultivée à 43°C en anaérobie sur milieu M17 liquide. La pureté des souches était contrôlée par la réalisation d'isolements (méthode des quadrants, Norme FIL 146 :1991). Le suivi de la population bactérienne était effectué via des dénombrements (Norme FIL 117B :1997) sur gélose MRS pH 5,4 pour *L. bulgaricus* et sur gélose M17 lactose pour S. *thermophilus.* La veille de la préparation de yaourt, des dilutions (en eau peptonée) de chacune des souches étaient ensemencées dans 10 ml de lait autoclavé. Après incubation 1 nuit à 43°C, la dilution n'ayant pas provoqué la coagulation du lait était choisie comme pré-culture.

### Fermentation du lait par culture mixte

Yaourt préparé au laboratoire: 100 ml de lait UHT écrémé du commerce additionnés de 2 % (p/p) de poudre de lait écrémé Ultra Low Heat (INRA Rennes) étaient incubés 45 min à 37°C sous agitation, puis subissaient un traitement thermique de 10 min à 90°C. Après une redescente en température à 43°C, le lait était ensemencé par 2 g de préculture de chacun des ferments. Le milieu était ensuite incubé à 43°C jusqu'à atteindre un pH de 4,9 - 4,5 au bout de 2 à 5 h. L'acidification du milieu était suivie au pH-mètre (poste multiple, CINAC) et un dénombrement bactérien était effectué à t0 (inoculation) et à tf. Le yaourt obtenu était stocké à 4°C et l'évolution de la protéolyse était suivie par dosage de la MAT (Matière Azotée Totale) et par RP-HPLC analytique (Reverse Phase-High Performance Liquid Chromatography) jusqu'à 3 semaines de stockage (prélèvements à 24 h, au 10^{ème} jour et au 20^{ème} jour).

Yaourt du commerce : le yaourt du commerce utilisé était un yaourt nature dont les valeurs nutritionnelles moyennes pour 100 g de produit étaient les suivantes (indications fabricant) : 4,1 % de protéines, 5,5 % de glucides, 1 % de lipides. En conditions standard, ce type de yaourt est issu de la fermentation par les bactéries lactiques d'un lait supplémenté en protéines et traité thermiquement dont la teneur en matière grasse a été ajustée.

### Obtention du pool peptidique total du yaourt

Le yaourt était homogénéisé manuellement puis centrifugé 30 min à 5000 g à température ambiante. Le surnageant obtenu était ensuite filtré sur papier Whatman n° 41. A ce stade, une précipitation sélective des protéines du surnageant était obtenue par l'ajout de TCA (acide trichloroacétique) à 2 % final, ceci afin d'éliminer les protéines du lactosérum (majoritairement β-lactoglobuline et α-lactalbumine). Après 30 minutes, une centrifugation de 10 min à 5000 g permettait d'obtenir le surnageant correspondant au pool peptidique total du yaourt. Une étape finale de dialyse extensive contre de l'eau désionisée (tubes SpectraPor, membrane en ester de cellulose, seuil de coupure 500 Da) permettait d'éliminer le lactose, le TCA et de diminuer la force ionique des échantillons en vue de la culture cellulaire.

### Obtention des fractions peptidiques du yaourt

Les fractions peptidiques du yaourt ont été obtenues par RP-HPLC préparative. Préalablement à l'injection, les pools peptidiques totaux étaient filtrés sur 1,2 µm et la colonne était équilibrée pendant 30 min avec du tampon A. La colonne utilisée était une colonne préparative (250 × 50 mm) contenant un support greffé en C18 (C18, LiChrospher RP18, 12 µm, Merck). Le tampon A était constitué de TFA 1,06 ‰ (acide trifluoroacétique) dans de l'eau milliQ et le tampon B de TFA 1 ‰ dans de l'acétonitrile à 80 %. Après 18 min d'élution en isocratique sous tampon A, l'élution était réalisée à 45 ml/min en 4 plateaux d'élution successifs de 8 min à 15 %, 30 %, 45 % puis 60 % de tampon B. Un plateau final de 20 min à 100 % de tampon B terminait le gradient. La détection était réalisée à 214 nm, avec une collecte manuelle des fractions. Après 2 injections de 60 ml d'échantillon, les fractions correspondantes étaient poolées puis évaporées sous vide (Rotavapor, Buchi) afin d'éliminer l'acétonitrile et la majorité de l'eau. Les échantillons étaient ainsi concentrés jusqu'à l'obtention d'environ 10 ml de produit, ajustés à pH 7 avec de l'ammoniaque (25 %) puis lyophilisés.

### Caractérisation des peptides du yaourt

### - Dosage de la matière azotée totale (MAT) par micro-Kjeldhal

Le dosage de la MAT était réalisé par la méthode de Kjeldhal (Ogg (1960) J. Assoc. Off. Anal. Chem. 43:689-694) appliquée à l'analyse des petits volumes soit 500 µl. Une gamme étalon de sulfate d'ammonium était réalisée pour chaque analyse. Les résultats étaient exprimés en mg d'azote/ml. Les échantillons sont analysés en dupliquas. Le taux optimal de récupération de l'azote se situait entre 97 et 99 %, caractérisé par une très bonne reproductibilité.

### - Chromatographie liquide haute performance en phase inversée (RP-HPLC analytique)

La chromatographie était réalisée sur une chaîne Waters (Waters alliance). L'absorbance était suivie à 214 et 280 nm. Les colonnes utilisées étaient une Symmetry C18 (150 × 2,1 mm) pour l'analyse des peptides et une Vydac C4 (150 × 4,6 mm) pour l'analyse des protéines. Pour l'analyse sur Vydac C4, les échantillons étaient préalablement traités au DTT (Dithiotreitol, Sigma). L'élution était réalisée à 40°C après injection de 100 µl d'échantillon. Le tampon A était constitué de TFA 1,06 ‰ dans de l'eau milliQ et le tampon B de TFA 1 ‰ dans de l'acétonitrile à 80 %.

### - Nano-chromatographie liquide couplée à la spectrométrie de masse (Nano-LC-MS/MS)

Pour l'analyse en spectrométrie de masse (QSTAR XLR, Applied Biosystems), les échantillons préalablement préparés pour la RP-HPLC étaient dilués 20 fois dans du TFA 1 ‰ puis 5 µl étaient injectés à 200 nl/min pour l'analyse. La colonne utilisée (diamètre interne 75 µm, longueur 15 cm) était remplie avec une phase inverse C18. Les séquences en acides aminés des peptides étaient déterminées grâce aux bases de données accessibles via Internet (Mascot).

### RESULTATS

### Protéolyse dans le yaourt

### - Evolution du pH

L'association des deux ferments *Streptococcus salivarius* spp. *Thermophilus* et *Lactobacillus delbrueckii* spp. *Bulgaricus* provoque une acidification rapide du milieu qui atteignait des pHs autour de 4,9 et 4,5 après respectivement 3 et 4 h d'incubation.

### - Evolution de la MAT

Un lait UHT écrémé du commerce supplémenté par 2 % de poudre de lait écrémé Ultra Low Heat (INRA Rennes) était utilisé pour la préparation de yaourt. Les yaourts obtenus étaient stockés à 4°C et l'évolution de la protéolyse était suivie pendant 3 semaines grâce à des dosages à 24 h, 10 jours et 20 jours de stockage. Une augmentation moyenne de 0,5 % des valeurs de MAT était observée au cours des 3 semaines de stockage. Cependant le suivi des échantillons par HPLC analytique effectué en parallèle montre qu'aucun changement n'intervient sur le plan qualitatif.

### Identification du pool peptidique du yaourt

### - Préparation et caractérisation par RP-HPLC

La combinaison des techniques de précipitation sélective au TCA 2 %, de dialyse sur membrane de 500 Da et de chromatographie a permis d'isoler et de purifier le pool peptidique à partir du milieu complexe qu'est le yaourt. Ce pool peptidique résultait de la protéolyse réalisée dans le yaourt par les ferments lactiques. Il était caractérisé par RP-HPLC, avec une élution selon une échelle d'hydrophobicité croissante obtenue grâce à un gradient linéaire d'acétonitrile.

*Yaourt du commerce* : Le profil d'élution du pool peptidique issu du yaourt du commerce était déterminé par RP-HPLC (indiqué par Pool peptidique C dans la **FIG. 1**). La première moitié du chromatogramme ainsi que les massifs retrouvés entre 40 et 53 min d'élution sont de nature peptidique. Le pic élué à 62 min est le résidu de β-lactoglobuline présent initialement dans le milieu avant l'étape de précipitation au TCA 2%.

*Yaourt préparé au laboratoire* : Le profil d'élution du pool peptidique issu du yaourt préparé au laboratoire était déterminé par RP-HPLC (indiqué par Pool peptidique F dans la FIG. 2). L'intensité des pics obtenus reflétait un plus faible contenu en peptides dans ce yaourt lorsqu'on le compare au yaourt du commerce. Ce résultat concorde avec la protéolyse plus faible mesurée par la MAT dans le yaourt préparé au laboratoire. Le yaourt préparé au laboratoire possédait en outre une texture plus visqueuse que celle du yaourt du commerce. Cette caractéristique peut être due au fait que la souche de *Lb. bulgaricus* employée se soit révélée productrice d'exopolysaccharides.

### Identification des peptides par nano-LC-MS/MS

*Yaourt du commerce* : Une bonne centaine de peptides ont pu être identifiés dans le yaourt du commerce. La majorité, soit environ une soixantaine, provient de la caséine β, environ 20 peptides sont issus de la caséine κ, 15 peptides proviennent de la caséine αₛ₁ et 5 peptides sont issus de la caséine αₛ₂

Plusieurs peptides potentiellement porteurs d'activité opiacée ont été identifiés parmi lesquels un certain nombre de peptides de la caséine β : 8 peptides contiennent la séquence Tyr-Pro-Phe-Pro-Gly-Pro-Ile de la β-casomorphine-7 (fragment [60-66] de la caséine β) considérés comme des pro-β-casomorphines en raison de la présence du motif Tyr-X(n)-Phe ; 6 peptides contiennent la séquence Tyr-Pro-Val-Glu-Pro-Phe-Thr correspondant au fragment [114-120] de la caséine β. Cette séquence opioïde [114-120] issue de la caséine β, a été identifiée en 1999 (Jinsmaa *et al.* (*op. cit*.)). Il faut aussi souligner que parmi les peptides identifiés issus de la caséine β, plusieurs autres sont des précurseurs de peptides bioactifs notamment des séquences contenant la séquence Ala-Val-Pro-Tyr-Pro-Gln-Arg dotée d'activité d'inhibition de l'enzyme de conversion de l'angiotensine I (ACE), et donc potentiellement d'activité antihypertensive.

**Tableau 1 : Peptides identifiés par nano-LC-MS/MS dans le pool peptidique du yaourt du commerce.**

| **Région de la caséine β** | **Peptides identifiés** |
|---|---|
| Pro-β-casomorphine (60-66) | 54-65 |
| | 57-68 |
| | 57-72 |
| | 57-77 |
| | 57-91 |
| | 57-93 |
| | 59-72 |
| Opioïde (114-120) | 94-123 |
| | 100-123 |
| | 102-123 |
| | 106-119 |
| | 106-123 |
| | 108-123 |

*Yaourt préparé au laboratoire* : A l'instar du yaourt du commerce, la majorité des peptides retrouvés dans le yaourt préparé au laboratoire est issue de la caséine β soit 55 peptides, tandis qu'une trentaine sont issus de la caséine αₛ₁, une dizaine de la caséine αₛ₂ et 2 de la caséine κ. Plusieurs précurseurs de peptides opiacés sont également identifiés dans le yaourt préparé au laboratoire (**Tableau 2**) dont 7 peptides considérés comme des pro-β-casomorphines couvrant la région 60-66 de la caséine β et 15 peptides contenant le fragment potentiellement opioïde [114-120] de la caséine β. De nombreuses séquences pro-hypertensives sont aussi retrouvées dans le yaourt du laboratoire.

**Tableau 2 : Peptides identifiés par nano-LC-MS/MS dans le pool peptidique du yaourt préparé au laboratoire.**

| **Région de la caséine β** | **Peptides identifiés** | |
|---|---|---|
| Pro-β-casomorphine (60-66) | 53-72 | |
| | 57-68 | |
| | 57-72 | |
| | 57-77 | |
| | 57-87 | |
| | 57-91 | |
| | 59-91 | |
| Opioïde (114-120) | | 92-123 |
| | 94-119 | 94-123 |
| | 98-119 | 97-123 |
| | 100-119 | 100-123 |
| | 101-119 | 101-123 |
| | 102-119 | 102-123 |
| | 103-119 | 103-123 |
| | | 106-123 |
| | | 108-123 |

Pour ces deux types de yaourt, on constate une coupure spécifique après la glutamine en position 123 dans la séquence de la caséine β. Cette coupure est liée à la protéase de paroi de type PIII qui hydrolyse la caséine β de manière spécifique après la glutamine en position 123 et qui est retrouvée notamment chez *Lactococcus lactis* subsp. *Cremoris* (Visser et al. (1991) Appl Microbiol Biotechnol. 35(4):477-83. Les systèmes protéolytiques des lactocoques (modèle *Lactococcus lactis* subsp. *cremoris*) et des lactobacilles (modèle *Lb. delbrueckii*) sont très proches et expliquent donc l'hydrolyse de la liaison 123-124. Dans le yaourt, on peut donc supposer que *Lb. bulgaricus,* doté d'une activité protéasique supérieure à celle de *S*. *thermophilus,* est responsable de cette spécificité de coupure.

### Isolement de fractions peptidiques

### - Préparation et caractérisation par RP-HPLC préparative

Les fractions peptidiques du yaourt ont été obtenues par RP-HPLC préparative à partir des pools peptidiques totaux comme décrits dans la partie Matériel et Méthodes.

*Fractions issues du yaourt du commerce* : 5 fractions ont été obtenues à partir du pool peptidique (**FIG. 1**). Ces fractions ont dénommées C1 à C5. La fraction C1, éluée pendant le premier plateau à 15 % de tampon B, n'a pu être testée sur le plan biologique car recueillie en trop faible quantité.

Les fractions C2, C3, C4 et C5 étaient éluées respectivement à 30, 45, 60 et 100 % de tampon B.

*Fractions issues du yaourt préparé au laboratoire* : 5 fractions ont été obtenues à partir du pool peptidique (FIG. 2). De la même manière que pour C1 dans le yaourt du commerce, la fraction F1 éluée pendant le plateau à 15 % de tampon B n'a pas pu être testée car récupérée en trop faible quantité.

Les fractions F2, F3, F4 et F5 sont respectivement éluées au cours des plateaux à 30, 45, 60 et 100 % de tampon B.

### - Identification des peptides par nano-LC-MS/MS

*Fractions issues du yaourt du commerce* : Les peptides d'intérêt identifiés dans les fractions C2, C3 et C4 sont présentés en tableau 3. Trois peptides contenant la séquence [114-120] de la caséine β sont identifiés dans la fraction C2 dont le peptide [94-123]. La fraction C3 contient 5 précurseurs de la séquence [114-120] de la caséine β ainsi que 3 pro-β-casomorphines, de séquences [57-68], [57-72] et [59-72]. La fraction C4, quand à elle, contient 3 pro-β-casomorphines et la fraction C5 contient 5 peptides issus de la caséine β, pour lesquels aucune séquence d'intérêt n'a été identifiée.

**Tableau 3 : Peptides identifiés par nano-LC-MS/MS dans les différentes fractions issues du yaourt du commerce.**

| Peptides identifiés | Fraction | | |
|---|---|---|---|
| | C2 | C3 | C4 |
| 54-65 | | | |
| 57-68 | | + | |
| 57-72 | | + | |
| 57-77 | | | + |
| 57-91 | | | + |
| 57-93 | | | + |
| 59-72 | | + | |
| 61-72 | | | |
| 94-123 | + | | |
| 100-123 | | + | |
| 102-123 | + | + | |
| 106-119 | | + | |
| 106-123 | + | + | |
| 108-123 | | + | |

*Fractions issues du yaourt préparé au laboratoire* : Les fractions F3 et F4 contiennent des peptides d'intérêt. Aucun peptide issu de la caséine β n'est identifié dans la fraction F2, tandis que la fraction F5 ne contient pas de peptides d'intérêt. La fraction F3 contient 2 pro-β-casomorphines de séquences [57-68], [57-72], et 6 précurseurs de la séquence opioïde [114-120] ayant tous la glutamine123 en position Cₜₑᵣₘᵢₙₐₗₑ. La fraction F4 contient 5 pro-β-casomorphines et 6 dérivés de la séquence opioïde [114-120] ayant tous la phenylalanine119 en position Cₜₑᵣₘᵢₙₐₗₑ.

**Tableau 4 : Peptides identifiés par nano-LC-MS/MS dans les différentes fractions issues du yaourt préparé au laboratoire.**

| Peptides identifiés | Fraction | |
|---|---|---|
| | F3 | F4 |
| 53-72 | | |
| 57-68 | + | |
| 57-72 | + | + |
| 57-77 | | + |
| 57-87 | | + |
| 57-91 | | + |
| 59-91 | | + |
| 92-123 | | |
| 94-119 | | + |
| 94-123 | + | |
| 97-123 | | |
| 98-119 | | + |
| 100-119 | | + |
| 100-123 | + | |
| 101-119 | | + |
| 101-123 | | |
| 102-119 | | + |
| 102-123 | + | |
| 103-119 | | + |
| 103-123 | + | |
| 106-123 | + | |
| 108-123 | + | |

### EXEMPLE 2 : Les peptides présents dans les laits fermentés modulent in vitro la sécrétion et l'expression de mucines gastro-intestinales

### MATERIEL ET METHODE

### Culture cellulaire

Les cellules HT29-MTX et HT29-CI16E étaient cultivées en flasques de 25 cm² avec du milieu minimum essentiel DMEM supplémenté avec 10% de SVF (sérum de veau foetal) et 1 % d'antibiotiques (pénicilline 10 000 Ul/ml et streptomycine 10 µg/ml). Les cellules étaient incubées à 37°C dans une atmosphère humide (5 % de CO₂ et 95 % d'O₂). A confluence, les cellules soumises au préalable à l'action de la trypsine (trypsine-EDTA) étaient cultivées dans des plaques 12 puits à raison de 1 ml de milieu de culture complet par puits. Afin de maintenir les cellules dans des conditions optimales de croissance, le milieu de culture était changé tous les 2 jours.

### Protocoles expérimentaux

Les expériences ont été réalisées sur les plaques 12 puits 21 jours après confluence des cellules. La veille de la stimulation, les plaques étaient cultivées sans SVF. Le pool peptidique total des yaourts ainsi que les différentes fractions lyophilisées étaient d'abord repris dans de l'eau stérile en solution 10 fois concentrée (10[Y]), [Y] correspondant à la concentration des peptides dans le yaourt initial). L'osmolarité et le pH des échantillons (ajusté à la neutralité par NaOH) étaient contrôlés à ce stade. Des dilutions successives ([Y], [Y]/2 et [Y]/5) étaient ensuite réalisées dans du milieu de culture dépourvu de SVF. Le jour de l'expérience, le milieu était aspiré puis les cellules étaient lavées avec du PBS à 37°C. Du milieu sans SVF contenant ou non (contrôles) les fractions peptidiques du yaourt à la concentration souhaitée était déposé à raison de 1 ml par puits (triplicata). Les cellules étaient alors incubées 4 h à 37°C en atmosphère humide. Les surnageants de culture étaient ensuite collectés et congelés à -20°C en vue du dosage des mucines par ELLA (Enzyme Linked Lectin Assay). Les cellules étaient enfin lysées par l'ajout de Tri-Reagent et leur contenu en acides nucléiques était prélevé en vue de l'extraction des ARN totaux. Chaque stimulation était réalisée à 4 reprises.

### Estimation du niveau d'ARNm par RT-PCR semi-quantitative

Les ARN ont été extraits à l'aide deTri-Reagent puis rétro-transcrits à l'aide du kit « RevertAid H Minus First Strand cDNA Synthesis Kit » en utilisant des amorces nucléotidiques au hasard (random hexamer). L'amplification était réalisée à l'aide de Taq polymérase (Invitrogen) et d'amorces spécifiques recherchées sur internet à l'aide des sites spécialisés ou issues de la littérature. La cyclophiline A a été utilisée comme gène de ménage. Après un nombre de cycles déterminé, choisi de façon à rester dans la phase exponentielle d'amplification, la réaction était arrêtée à +4°. Les échantillons étaient alors analysés par migration en électrophorèse sur gel d'agarose imprégné de bromure d'éthidium pour la révélation des ADNc. Les taches étaient visualisées sous lumière ultraviolette et pixelisées à l'aide d'un tans-illuminateur (Quantum Appligene). L'analyse d'image a été réalisée à l'aide du logiciel Scion image.

### Mesure du niveau d'ARNm par RT-PCR quantitative sur appareil LightCycler (Roche)

Les ARN ont été isolés et rétro-transcrits comme décrit précédemment puis les ADNc ont été quantifiés en capillaires à l'aide du kit « FastStart DNA Master SYBR Green l » sur un appareil « lightCycler » (Roche). La cyclophiline A a été utilisée comme gène de ménage et les calculs ont été réalisés par la méthode « DDCt »

### RESULTATS

### Effets des pools peptidiques totaux sur la sécrétion des mucines

L'activité biologique des deux pools peptidiques a été recherchée dans la lignée mucosécrétante intestinale humaine HT29-MTX. Pour cela, les cellules étaient exposées à différentes concentrations de pool peptidique pendant 4 h, C désignant le yaourt du commerce et F le yaourt fait au laboratoire. La concentration Y correspond à la MAT des peptides dans le yaourt initial. L'osmolarité était ajustée à 330 mmosml/L et le pH à 7. Au cours de ces expériences, il a été montré que la sécrétion de mucines était augmentée de manière dose dépendante par les pools peptidiques totaux issus des deux yaourts. Cet effet était maximum à la concentration Y (**FIG. 3**).

### Effets des pools peptidiques totaux sur l'expression des mucines

L'étude a porté sur les deux principales mucines gastro-intestinales secrétées produites par les cellules HT29-MTX, les mucines MUC2 et MUC5AC, ainsi que sur la mucine membranaire MUC4. Dans le tractus intestinal, cette mucine est également décrite comme pouvant être produite sous une forme secrétée (Rong et al. (2005) J Cell Physiol 202(1):275-84). Elle pourrait donc participer aux propriétés du gel de mucus. La stimulation de la lignée HT29-MTX pendant 4 h par le pool peptidique C (concentration Y, Y/2 et Y/5) s'est traduite par une augmentation du niveau des transcrits codant MUC2 et MUC4 par rapport aux cellules non traitées alors que les transcrits de MUC5AC n'étaient pas modifiés de façon significative (**FIG. 4** ; analyse par RT-PCR semi-quantitative). Le pool peptidique F a augmenté l'expression de MUC5AC et MUC4, avec une réponse maximale observée à la concentration Y. Dans nos expériences, les pools peptidiques totaux C et F n'ont pas modifié l'expression de la cyclophiline (gène de ménage).

### Effets des différentes fractions C du yaourt du commerce sur la sécrétion et l'expression de mucines in vitro (HT29-MTX)

### - Effets des fractions C sur la sécrétion des mucines

Suite à ce résultat, les fractions C2, C3, C4 et C5 (à la concentration Y), obtenues par chromatographie préparative (RP-HPLC), ont été testées sur la sécrétion et l'expression des mucines gastro-intestinales. Le dosage des surnageants de culture par ELLA a permis de montrer que seule la fraction C2 induit une augmentation significative de la sécrétion de mucines par rapport aux cellules non traitées (203+/-11 % des contrôles, p<0,05) (**FIG. 5**). Cette fraction a un effet dose dépendant sur la sécrétion de mucines (**FIG. 6**) et sur le niveau des transcrits codant MUC2 et MUC4 (**FIG. 7** et **FIG. 8** ; analyse par RT-PCR semi-quantitative) par rapport aux cellules non traitées. En revanche, l'expression de MUC5AC n'était pas modifiée. Parmi les peptides identifiés dans les différentes fractions, seul le [94-123] est présent dans la fraction active (C2) tout en étant absent des autres fractions, ce qui en fait un bon candidat pour être responsable de l'activité biologique (cf Tableau 3).

### Effets des différentes fractions F du yaourt du laboratoire sur la sécrétion et l'expression de mucines in vitro (HT29-MTX)

### - Effets des fractions F sur la sécrétion des mucines

Les fractions F3, F4 et F5 (à la concentration Y) obtenues par chromatographie préparative (RP-HPLC) ont été testées sur la sécrétion et l'expression des mucines gastrointestinales. Aucun peptide issu de la caséine β n'ayant été identifié dans la fraction F2, cette dernière n'a pas été testée. La fraction F3 a induit une augmentation significative de la sécrétion de mucines par rapport aux cellules non traitées (160 +/-8 % des contrôles, p<0,05). En revanche, les fractions F4 et F5 n'ont montré aucun effet sur la sécrétion de mucines.

La fraction F3 contient 2 pro-β-casomorphines (peptides [57-68] et [57-72]) et 6 précurseurs de la séquence opioïde [114-120]. De même que pour le yaourt du commerce, le peptide de séquence [94-123] a été identifié dans la fraction active, ce qui conforte l'hypothèse d'un effet biologique induit par ce peptide [94-123].

### - Effet dose de la fraction F3 sur la sécrétion de mucines

Une étude dose réponse a été réalisée pour la fraction F3. Les résultats obtenus ont montré que cette fraction a un effet dose dépendant sur la sécrétion de mucines.

### - Effets de la fraction F3 sur l'expression des mucines

Contrairement aux résultats obtenus avec la fraction C2, la stimulation des cellules HT29- MTX par la fraction F3 pendant 4 h n'a pas modifié le niveau des transcrits codant MUC2 par rapport aux cellules non traitées. En revanche, une augmentation significative du niveau des transcrits codant MUC5AC et MUC4 était observée après incubation des cellules en présence de cette fraction.

En conclusion, l'effet de chaque pool peptidique (C et F) a été reproduit par une seule de ses fractions (respectivement C2 et F3). Les deux fractions actives contiennent des précurseurs de peptides opioïdes ainsi que le peptide (94-123). La fraction C2 a augmenté l'expression des mucines gastro-intestinales MUC2 et MUC4 alors que la fraction F3 augmentait celle de MUC5AC et MUC4.

### Mise en jeu de la voie µ-opioïdergique par les fractions C2 et F3

Des récepteurs µ opioïdergiques ont été mis en évidence à la surface des cellules HT29-MTX (Zoghbi *et al.* (*op. cit*.)). La présente étude montre que la pré-incubation des cellules HT29-MTX en présence de cyprodime (10⁻⁵ M), un antagoniste spécifique du récepteur µ-opioïdergique (Marki et al. (1999) Eur J Pharmacol 383(2) :209-14), inhibe la sécrétion de mucines induite par les fractions C2 (**FIG. 9**) ou F3 (concentration Y). L'effet de la fraction C2 sur l'expression des mucines MUC2 et MUC4 était également aboli par l'utilisation de cet antagoniste. Par RT-PCR quantitative sur appareil Lightcycler, il a également été montré que le cyprodime abolit l'augmentation du niveau des transcrits codant MUC5AC induite par la fraction F3. Un résultat identique a été observé avec la mucine MUC4.

Ces résultats permettent de penser que les fractions C2 et F3 exercent leur effet sur la sécrétion et sur l'expression des mucines via l'activation des récepteurs µ-opioïdes.

Les deux fractions d'intérêt C2 et F3 ont également été testées à la concentration Y dans une autre lignée mucosécrétante, la lignée HT29-CI16E. Les résultats obtenus sur l'expression de MUC2, MUC5AC et MUC4 étaient similaires à ceux observés sur les cellules HT29-MTX avec une augmentation de l'expression des mucines MUC2 et MUC4 sous stimulation par la fraction C2 (4 h, concentration Y) et une augmentation de l'expression des mucines MUC5AC et de MUC4 sous stimulation par la fraction F3 (4 h, concentration Y). Le dosage des surnageants de culture par ELLA a montré également que les fractions C2 et F3 augmentent de façon significative la quantité de mucines libérées dans le milieu de culture après 4 h de stimulation (172+/- 3% et 157+/-8% pour les fractions CE et F3, respectivement, p<0,05).

### Effet du peptide [94-123], un précurseur de la néocasomorphine identifié dans les deux fractions C2 et F3

La β-casomorphine-7, peptide µ-opioïdergique exogène de référence, exerce un effet biologique sur les cellules HT29-MTX à la dose de 10⁻⁴ M en augmentant notamment la sécrétion de mucines et l'expression de MUC5AC. Le peptide [94-123] (Millegen, Labege, France) testé à cette même concentration, a en revanche, inhibé la sécrétion de mucines (**FIG. 10**) ainsi que l'expression de MUC4 (**FIG. 12**) mesurée par RT-PCR quantitative sur appareil Lightcycler. Il est à noter que ce peptide induit une augmentation significative des transcrits codant MUC2 lorsqu'il est testé à la concentration de 10⁻⁸, 10⁻⁷ et 10⁻⁶ M (**FIG. 11**). D'autres résultats ont permis de montrer qu'à la concentration de 10⁻⁸ et 10⁻⁷ M, ce peptide provoque également une forte augmentation de l'expression de la mucine MUC4. Dans toutes nos expériences, le peptide [94-123] (10⁻⁸-10⁻⁴M) était sans effet sur l'expression de MUC5AC.

Nous avons également montré que le peptide **[94-123] n'agissait pas par la voie mu-opioidergique.**

### EXEMPLE 3 : Le peptide [94-123] module in vivo l'expression de mucines gastro-intestinales et du lysozyme et modifie le nombre de cellules à mucus et de cellules de Paneth.

Le peptide [94-123] a été administré seul par gavage à de jeunes rats afin d'évaluer son impact sur l'expression de la mucine sécrétée (MUC2) et de la mucine membranaire (MUC4) dans le duodénum, le jéjunum, l'iléon et le côlon. L'effet du traitement sur les cellules à mucus (production de MUC2) et les cellules de Paneth (cellules ayant rôle fondamental dans la défense antimicrobienne) a également été évalué par immunohistochimie et qRT-PCR.

### MATERIEL ET METHODE

### Expériences animales

Le peptide [94-123] a été administré une fois par jour par canulation gastrique à de jeunes rats (Wistar, Harlan, France) du 10^{ème} au 18^{ème} jour postnatal. Les concentrations en peptide administrées étaient de 10⁻⁸, 10⁻⁷, 10⁻⁶ ou 10⁻⁴ M sous un volume de 900 µl/100 g de poids. Les animaux contrôles recevaient le véhicule seul (eau). Chaque groupe expérimental était constitué de 9 ratons.

### Analyse histologique

Après sacrifice des animaux, des échantillons de duodénum, de jéjunum, d'iléon, de côlon proximal et de côlon distal étaient prélevés et fixés dans l'alcool à 80 % en vue de l'analyse histologique. Des biopsies (longueur : 1 cm) étaient également congelées dans de l'azote liquide en vue de l'extraction des ARNm.

### Techniques immunohistochimiques

Des coupes (5 µm) d'intestin inclus en paraffine ont été successivement déparaffinées, réhydratées et incubées en présence de peroxyde d'hydrogène (3% dans du PBS, 20 min). Le traitement des lames aux microondes a permis le démasquage des sites antigéniques. Après saturation des sites non spécifiques par une incubation dans du sérum de cheval dilué à 10 % dans du PBS, les coupes étaient incubées pendant une heure en présence de l'anticorps primaire (MUC2, H-300, Santacruz, 1/250 ou Lysozyme, Zymed Laboratories, 1/100) dilué dans du PBS contenant 1 % de sérum de cheval. Pour l'immunohistochimie du lysozyme, l'étape de démasquage au microonde n'était pas réalisée. Après rinçages, les coupes étaient incubées en présence de l'anticorps secondaire couplé à la peroxydase (ImmPress, Vector laboratories, Abcys), puis du réactif de révélation (AEC, Vector laboratories, Abcys). Les coupes étaient alors contre-colorées (hématoxyline, Vector laboratories) et montées en milieu aqueux.

### Mesure du niveau d'ARNm par RT-PCR quantitative sur appareil Mastercycler ep Realplex (Eppendorf)

Les ARN ont été isolés et rétro-transcrits comme décrit dans l'exemple 2. L'amplification était réalisée sur plaque de 96 puits à l'aide d'un kit pour PCR quantitative (Maxima SYBR Green qPCR Master Mix, Fermentas) et d'amorces spécifiques recherchées sur internet à l'aide des sites spécialisés ou issues de la littérature. La cyclophiline A a été utilisée comme gène de ménage et les calculs ont été réalisés par la méthode « DDCt »

### RESULTATS

### Effet du peptide [94-123] sur les cellules à mucus.

Dans l'exemple 2, il a été montré *in vitro* sur la lignée intestinale mucosécrétante humaine HT29-MTX que, la séquence [94-123] augmente fortement l'expression de MUC2 ou/et de MUC4 lorsqu'il était testé à des concentrations comprises entre 10⁻⁸ et 10⁻⁶ M. Un tel effet se retrouve dans l'intestin grêle de rat. En effet, le suivi des transcrits par qRT-PCR a permis de montrer que le peptide (administré par gavage aux concentrations 10⁻⁷ et 10⁻⁴ M) induit l'expression de MUC2 dans le jéjunum de rat (Tableau 5).

En immunohistochimie, cet effet se traduit par une augmentation des cellules caliciformes dans le duodénum (10⁻⁷M), le jéjunum (10⁻⁷ et 10⁻⁴ M) et l'iléon (10⁻⁷ et 10⁻⁴ M), suggérant un impact possible sur la différenciation de cette lignée cellulaire (Tableau 5 et FIG. 13).

Il est à noter que l'expression de la mucine MUC4 est également augmentée par le peptide le long du tractus gastro-intestinal (Tableau 5) mais uniquement lorsque ce dernier est testé aux concentrations de 10⁻⁸ et 10⁻⁴ M.

**Tableau 5 : Effet de l'administration par sonde gastrique (J10-J18) du peptide [94-123] (β-CN (94-123)) sur le nombre de cellules à mucus par axe crypto-villositaire et sur l'expression des mucines MUC2 et MUC4 dans le duodénum, le jéjunum et l'iléon de rat. Les résultats de qRT-PCR sont exprimés en % des CT. * p<0.05 vs véhicule. Les données ont été analysées par une ANOVA à un facteur suivie du test de Bonferroni**

| | MUC2 | Immunohistochimie | | | MUC2 | qRT-PCR | | MUC4 | qRT-PCR | |
|---|---|---|---|---|---|---|---|---|---|---|
| | Duodénum | Jéjunum | iléon | | Duodénum | Jéjunum | iléon | Duodénum | Jéjunum | iléon |
| CT | 6,75 (0,11) | 7,75(0,14) | 8,03 (0,14) | | 100 (1,3) | 100 (1,2) | 100 (1,7) | 100 (1,7) | 100 (2,8) | 100 (2,1) |
| **β-CN(94-123) 10-8M** | 6,57 (0,23) | 7,96 (0,18) | 7,61 (0,18) | | 83 (0,8) | 102 (1,8) | 98 (1,35) | 144 (1,3)* | 160 (1,4)* | 157 (2,2) * |
| **β-CN(94-123) 10-7M** | 8,86 (0,17)* | 9,22 (0,24)* | 9,79 (0,09)* | | 84 (1,3) | 152 (1,1)* | 104 (1,1) | 112 (1,5) | 94 (1,2) | 84 (0,7) |
| **β-CN(94-123) 10-4M** | 6,61 (0,16) | 10,21 (0,21)* | 10,54 (0,17)* | | 90 (1,0) | 139 (1,8)* | 84(1) | 106 (1,4) | 161 (2,1)* | 113 (1,7) |

### Effet du peptide [94-123] sur les cellules de Paneth

Les cellules de Paneth jouent un rôle majeur dans la défense intestinale. Ces cellules sont uniquement localisées dans le bas des glandes de Lieberkühn de l'intestin grêle. Elles expriment la mucine MUC4 et se caractérisent par la présence, à leur pôle apical, de micro-granules de sécrétion contenant notamment du lysozyme et des α-défensines (peptides naturels à vocation d'antibiotiques).

L'activité biologique du peptide [94-123] a été recherchée sur les cellules de Paneth grâce à des techniques immunohistochimiques ciblant le lysozyme. Chez le rat, les cellules de Paneth apparaissent dans l'intestin grêle entre J10 et J13 postnatal. A J18 postnatal, les cellules de Paneth s'observent dans environ 20 % des cryptes du duodénum. Nos résultats montrent que le peptide [94-123], administré par gavage à la concentration de 10⁻⁷, 10⁻⁶ M et 10⁻⁴ M, augmente fortement le pourcentage de cryptes contenant des cellules de Paneth (p<0.05) dans le duodénum (**FIG. 14**).

Les données obtenues par qRT-PCR nous ont également permis de montrer que le peptide [94-123] augmente le niveau des transcrits codant le lysozyme (Tableau 6).

Ces effets sur les cellules de Paneth et l'expression du lysozyme obtenus suite à l'administration du peptide s'observent également dans le jéjunum et l'iléon (Tableau 6, **FIG. 15** et **FIG. 16**). Dans ces deux parties du tractus, la première réponse significative est observée lorsque le peptide est donné à la concentration de 10⁻⁸ M.

Le peptide [94-123] ou une forme issue de sa digestion résisterait donc à l'action des enzymes du tractus intestinal et parviendrait jusqu'à l'iléon sous une forme active. De tels peptides pourraient jouer un rôle fondamental dans le maintien de la fonction de barrière et la défense intestinale, notamment en période postnatale. Il est cependant à remarquer qu'un effet indirect est également possible.

**Tableau 6: Effet de l'administration par sonde gastrique (J10-J18) du peptide [94-123] sur le pourcenta0ge de cryptes montrant des cellules de Paneth et sur l'expression du lysozyme dans le duodénum, le jéjunum et l'iléon de rat. Les résultats de qRT-PCR sont exprimés en % des CT. * p<0.05 vs véhicule. Les données ont été analysées par une ANOVA à un facteur suivie du test de Bonferroni.**

| | Lysozyme | Immunohistochimie | | | Lysozyme | qRT-PCR | |
|---|---|---|---|---|---|---|---|
| | Duodénum | Jéjunum | iléon | | Duodénum | Jéjunum | iléon |
| **CT** | 21,39 (0,6) | 45,65 (0,49) | 43,09 (1,26) | | 100 (0,9) | 100 (1) | 100 (1,4) |
| **[94-123] 10-8M** | 28,51 (0,94) | 59,29 (0,76) * | 59,38 (0,58) * | | 82 (1,2) | 118 (2) | 113 (2) |
| **[94-123] 10-7M** | 34,53 (0,77) * | 51,92 (0,44) | 49,8 (1,08) | | 139 (1,8)* | 110 (2) | 101 (1) |
| **[94-123] 10-4M** | 37,26 (0,63) * | 66,9 (0,45) * | 61,19 (1,23) * | | 128 (1,9)* | 140 (1,5) * | 128 (1,5) |

L'étude *in vivo* a révélé un effet important du peptide [94-123] sur deux populations de cellules cruciales pour l'immunité innée et la défense intestinale : les cellules à mucus et les cellules de Paneth. L'impact du peptide [94-123] sur le nombre de cellules de Paneth pourrait rendre notamment la muqueuse intestinale moins vulnérable aux infections bactériennes. Il est à noter que le peptide est actif à faible concentration (10⁻⁸ M dans le jéjunum et l'iléon, 10⁻⁷ M dans le duodénum), concentration pouvant être présente dans les laits fermentés. Cette étude est également la première, à notre connaissance, à montrer une interaction positive entre un nutriment et les cellules de Paneth. La biologie et les facteurs concourant à la maturation des cellules de Paneth ou à leur activité sécrétoire sont encore mal connus du fait notamment de l'absence de modèle d'étude cellulaire. L'effet du peptide [94-123] sur les cellules de Paneth représente donc un réel progrès en termes de valorisation dans un contexte d'alimentation préventive et de développement de produits innovants.

## Revendications

1. Polypeptide isolé constitué de :
- SEQ ID NO : 1, ou
- une séquence présentant au moins 90% d'identité avec SEQ ID NO : 1, ou
- une séquence d'au moins 20 acides aminés consécutifs comprise dans SEQ ID NO :1,
qui présente au moins un effet choisi parmi :
- l'induction de l'expression et/ou de la sécrétion d'au moins une mucine gastro-intestinale ; et
- l'induction de l'expression et/ou de la sécrétion d'au moins une molécule de défense intestinale exprimée par les cellules de Paneth ; et
- l'induction de l'augmentation de la population des cellules à mucus et/ou de la population des cellules de Paneth,
le pourcentage d'identité étant le pourcentage d'acides aminés identiques entre deux séquences à comparer, les comparaisons entre deux séquences polypeptidiques étant réalisées après avoir aligné ces deux séquences de manière optimale sur la totalité de leur longueur, le nombre de positions pour lesquelles les acides aminés sont identiques étant ensuite divisé par le nombre total d'acides aminés de la plus grande des deux séquences comparées et le pourcentage d'identité étant obtenu en multipliant le résultat obtenu par 100.

2. Polypeptide selon la revendication 1, constitué de SEQ ID NO : 1.

3. Polypeptide selon la revendication 1 ou 2, pour son utilisation comme médicament.

4. Polypeptide constitué de :
- SEQ ID NO : 1, ou
- une séquence présentant au moins 90% d'identité avec SEQ ID NO : 1, ou
- une séquence d'au moins quatre acides aminés consécutifs comprise dans SEQ ID NO :1,
qui présente au moins un effet choisi parmi :
- l'induction de l'expression et/ou de la sécrétion d'au moins une mucine gastro-intestinale ; et
- l'induction de l'expression et/ou de la sécrétion d'au moins une molécule de défense intestinale exprimée par les cellules de Paneth ; et
- l'induction de l'augmentation de la population des cellules à mucus et/ou de la population des cellules de Paneth,
pour son utilisation comme médicament dans le traitement d'un défaut d'expression et/ou de sécrétion des mucines gastro-intestinales et/ou d'une altération de la barrière muqueuse gastrointestinale chez un patient,
le pourcentage d'identité étant le pourcentage d'acides aminés identiques entre deux séquences à comparer, les comparaisons entre deux séquences polypeptidiques étant réalisées après avoir aligné ces deux séquences de manière optimale sur la totalité de leur longueur, le nombre de positions pour lesquelles les acides aminés sont identiques étant ensuite divisé par le nombre total d'acides aminés de la plus grande des deux séquences comparées et le pourcentage d'identité étant obtenu en multipliant le résultat obtenu par 100.

5. Polypeptide pour son utilisation selon la revendication 4 comme médicament dans la prévention ou le traitement d'une pathologie sélectionnée dans le groupe constitué de l'inflammation intestinale notamment chronique, d'une infection intestinale, d'ulcères gastrique ou duodénal notamment dus à une infection à *Helicobacter pylori,* de diarrhée, de constipation, de cancers intestinaux et coliques, et de mucites induites par la radiothérapie ou la chimiothérapie.

6. Polypeptide constitué de :
- SEQ ID NO : 1, ou
- une séquence présentant au moins 90% d'identité avec SEQ ID NO : 1, ou
- une séquence d'au moins quatre acides aminés consécutifs comprise dans SEQ ID NO :1,
qui présente au moins un effet choisi parmi :
- l'induction de l'expression et/ou de la sécrétion d'au moins une mucine gastro-intestinale ; et
- l'induction de l'expression et/ou de la sécrétion d'au moins une molécule de défense intestinale exprimée par les cellules de Paneth ; et
- l'induction de l'augmentation de la population des cellules à mucus et/ou de la population des cellules de Paneth,
pour son utilisation comme médicament dans la prévention ou le traitement d'une déplétion et/ou d'un dysfonctionnement des cellules de Paneth chez un patient,
le pourcentage d'identité étant le pourcentage d'acides aminés identiques entre deux séquences à comparer, les comparaisons entre deux séquences polypeptidiques étant réalisées après avoir aligné ces deux séquences de manière optimale sur la totalité de leur longueur, le nombre de positions pour lesquelles les acides aminés sont identiques étant ensuite divisé par le nombre total d'acides aminés de la plus grande des deux séquences comparées et le pourcentage d'identité étant obtenu en multipliant le résultat obtenu par 100.

7. Polypeptide pour son utilisation selon la revendication 6, comme médicament dans la prévention ou le traitement d'une pathologie choisie parmi une pathologie liée à un défaut d'expression et/ou de sécrétion d'une molécule de défense intestinale exprimée par les cellules de Paneth, une infection intestinale dues à des bactéries, virus, levures ou protozoaires, une altération du microbiote intestinal, l'entérocolite nécrosante du nourrisson, une diarrhée, la maladie de Crohn et autres inflammations intestinales et coliques.

8. Composition pharmaceutique comprenant un polypeptide tel que défini dans la revendication 1 ou 2 à titre de substance active, en association avec un véhicule pharmaceutiquement acceptable.

9. Utilisation d'un polypeptide isolé constitué de :
- SEQ ID NO : 1, ou
- une séquence présentant au moins 90% d'identité avec SEQ ID NO : 1, ou
- une séquence d'au moins quatre acides aminés consécutifs comprise dans SEQ ID NO :1,
qui présente au moins un effet choisi parmi :
- l'induction de l'expression et/ou de la sécrétion d'au moins une mucine gastro-intestinale ; et
- l'induction de l'expression et/ou de la sécrétion d'au moins une molécule de défense intestinale exprimée par les cellules de Paneth ; et
- l'induction de l'augmentation de la population des cellules à mucus et/ou de la population des cellules de Paneth,
pour la préparation d'une composition alimentaire,
le pourcentage d'identité étant le pourcentage d'acides aminés identiques entre deux séquences à comparer, les comparaisons entre deux séquences polypeptidiques étant réalisées après avoir aligné ces deux séquences de manière optimale sur la totalité de leur longueur, le nombre de positions pour lesquelles les acides aminés sont identiques étant ensuite divisé par le nombre total d'acides aminés de la plus grande des deux séquences comparées et le pourcentage d'identité étant obtenu en multipliant le résultat obtenu par 100.

10. Utilisation selon la revendication 9, pour la préparation d'une composition alimentaire lactée.

11. Composition alimentaire non lactée comprenant un polypeptide constitué de :
- SEQ ID NO : 1, ou
- une séquence présentant au moins 90% d'identité avec SEQ ID NO : 1, ou
- une séquence d'au moins quatre acides aminés consécutifs comprise dans SEQ ID NO :1,
qui présente au moins un effet choisi parmi :
- l'induction de l'expression et/ou de la sécrétion d'au moins une mucine gastro-intestinale ; et
- l'induction de l'expression et/ou de la sécrétion d'au moins une molécule de défense intestinale exprimée par les cellules de Paneth ; et
- l'induction de l'augmentation de la population des cellules à mucus et/ou de la population des cellules de Paneth,
dans laquelle le polypeptide est à une concentration comprise entre 0.001 et 10µM,
le pourcentage d'identité étant le pourcentage d'acides aminés identiques entre deux séquences à comparer, les comparaisons entre deux séquences polypeptidiques étant réalisées après avoir aligné ces deux séquences de manière optimale sur la totalité de leur longueur, le nombre de positions pour lesquelles les acides aminés sont identiques étant ensuite divisé par le nombre total d'acides aminés de la plus grande des deux séquences comparées et le pourcentage d'identité étant obtenu en multipliant le résultat obtenu par 100.

12. Composition alimentaire lactée comprenant un polypeptide constitué de :
- SEQ ID NO : 1, ou
- une séquence présentant au moins 90% d'identité avec SEQ ID NO : 1, ou
- une séquence d'au moins quatre acides aminés consécutifs comprise dans SEQ ID NO :1,
qui présente au moins un effet choisi parmi :
- l'induction de l'expression et/ou de la sécrétion d'au moins une mucine gastro-intestinale ; et
- l'induction de l'expression et/ou de la sécrétion d'au moins une molécule de défense intestinale exprimée par les cellules de Paneth ; et
- l'induction de l'augmentation de la population des cellules à mucus et/ou de la population des cellules de Paneth,
dans laquelle le polypeptide est à une concentration d'au moins 7 µM,
le pourcentage d'identité étant le pourcentage d'acides aminés identiques entre deux séquences à comparer, les comparaisons entre deux séquences polypeptidiques étant réalisées après avoir aligné ces deux séquences de manière optimale sur la totalité de leur longueur, le nombre de positions pour lesquelles les acides aminés sont identiques étant ensuite divisé par le nombre total d'acides aminés de la plus grande des deux séquences comparées et le pourcentage d'identité étant obtenu en multipliant le résultat obtenu par 100.

13. Composition alimentaire selon la revendication 11, dans laquelle le polypeptide est à une concentration égale à 0.01 µM.

14. Procédé de préparation d'un produit lacté fermenté par au moins une souche de *Lactobacillus delbrueckii* ssp. *bulgaricus* et/ou au moins une souche de *Streptococcus salivarus* spp. *thermophilus* comprenant une étape de détermination de la concentration d'un polypeptide constitué de :
- SEQ ID NO : 1, ou
- une séquence présentant au moins 90% d'identité avec SEQ ID NO : 1, ou
- une séquence d'au moins quatre acides aminés consécutifs comprise dans SEQ ID NO :1,
qui présente au moins un effet choisi parmi :
- l'induction de l'expression et/ou de la sécrétion d'au moins une mucine gastro-intestinale ; et
- l'induction de l'expression et/ou de la sécrétion d'au moins une molécule de défense intestinale exprimée par les cellules de Paneth ; et
- l'induction de l'augmentation de la population des cellules à mucus et/ou de la population des cellules de Paneth,
le pourcentage d'identité étant le pourcentage d'acides aminés identiques entre deux séquences à comparer, les comparaisons entre deux séquences polypeptidiques étant réalisées après avoir aligné ces deux séquences de manière optimale sur la totalité de leur longueur, le nombre de positions pour lesquelles les acides aminés sont identiques étant ensuite divisé par le nombre total d'acides aminés de la plus grande des deux séquences comparées et le pourcentage d'identité étant obtenu en multipliant le résultat obtenu par 100.

15. Procédé selon la revendication 14, dans lequel on laisse agir la souche de *Lactobacillus delbrueckii* ssp. *bulgaricus* et/ou la souche de *Streptococcus salivarus* spp. *thermophilus* jusqu'à ce qu'une valeur prédéterminée de la concentration du polypeptide soit atteinte.

## Patentansprüche

1. Isoliertes Polypeptid bestehend aus:
- SEQ ID NO : 1, oder
- einer Sequenz mit mindestens 90 % Identität mit SEQ ID NO : 1, oder
- einer Sequenz aus mindestens 20 aufeinanderfolgenden Aminosäuren umfasst in SEQ ID NO : 1,
welches mindestens einen Effekt aufweist, ausgewählt aus:
- Induktion der Expression und/oder der Sekretion mindestens eines gastrointestinalen Mucins; und
- Induktion der Expression und/oder der Sekretion mindestens eines Moleküls der Darmabwehr, exprimiert durch Paneth-Zellen; und
- Induktion der Erhöhung der Population von Mukuszellen und/oder der Population von Paneth-Zellen,
wobei der Prozentsatz der Identität der Prozentsatz der identischen Aminosäuren zwischen zwei zu vergleichenden Sequenzen ist, wobei die Vergleiche zwischen zwei Polypeptidsequenzen nach der Alignierung dieser zwei Sequenzen optimalerweise über ihre Gesamtlänge durchgeführt werden, wobei die Anzahl der Positionen, an denen die Aminosäuren identisch sind, anschließend durch die Gesamtzahl der Aminosäuren der größeren der beiden verglichenen Sequenzen dividiert wird und der Prozentsatz der Identität durch Multiplizieren des erhaltenen Ergebnisses mit 100 erhalten wird.

2. Polypeptid gemäß Anspruch 1, bestehend aus SEQ ID NO : 1.

3. Polypeptid gemäß Anspruch 1 oder 2 zur Verwendung als Medikament.

4. Polypeptid bestehend aus:
- SEQ ID NO : 1, oder
- einer Sequenz mit mindestens 90 % Identität mit SEQ ID NO : 1, oder
- einer Sequenz mit mindestens vier aufeinanderfolgenden Aminosäuren umfasst in SEQ ID NO: 1,
welches mindestens einen Effekt aufweist, ausgewählt aus:
- Induktion der Expression und/oder der Sekretion mindestens eines gastrointestinalen Mucins; und
- Induktion der Expression und/oder der Sekretion mindestens eines Moleküls der Darmabwehr, exprimiert durch Paneth-Zellen; und
- Induktion der Erhöhung der Population der Mukuszellen und/oder der Population der Paneth-Zellen,
für seine Verwendung als Medikament in der Behandlung eines Defekts in der Expression und/oder Sekretion gastrointestinaler Mucine und/oder einer Veränderung der gastrointestinalen Mukosa-Barriere bei einem Patienten,
wobei der Prozentsatz der Identität der Prozentsatz der identischen Aminosäuren zwischen zwei zu vergleichenden Sequenzen ist, wobei die Vergleiche zwischen zwei Polypeptidsequenzen vor der Alignierung dieser zwei Polypeptidsequenzen optimalerweise über ihre Gesamtlänge realisiert werden, wobei die Anzahl der Positionen, für die die Aminosäuren identisch sind, anschließend durch die Gesamtzahl der Aminosäuren der größeren der beiden verglichenen Sequenzen dividiert wird und der Prozentsatz der Identität durch Multiplizieren des erhaltenen Ergebnisses mit 100 erhalten wird.

5. Polypeptid für seine Verwendung gemäß Anspruch 4 als Medikament in der Prävention oder der Behandlung einer Erkrankung ausgewählt aus der Gruppe bestehend aus Darmentzündung, insbesondere chronischer, Darminfektion, Magen- oder Zwölffingerdarmgeschwüren, insbesondere aufgrund einer Infektion mit *Helicobacter pylori,* Diarrhö, Verstopfung, Darm- und Colonkarzinomen und durch Strahlentherapie oder Chemotherapie induzierte Mukositis.

6. Polypeptid bestehend aus:
- SEQ ID NO: 1, oder
- einer Sequenz mit mindestens 90 % Identität mit SEQ ID NO : 1, oder
- einer Sequenz aus mindestens vier aufeinanderfolgenden Aminosäuren umfasst in SEQ ID NO: 1,
welches mindestens einen Effekt aufweist, ausgewählt aus:
- Induktion der Expression und/oder der Sekretion mindestens eines gastrointestinalen Mucins; und
- Induktion der Expression und/oder der Sekretion mindestens eines Moleküls der Darmabwehr, exprimiert durch Paneth-Zellen; und
- Induktion der Erhöhung der Population von Mukuszellen und/oder der Population von Paneth-Zellen,
für seine Verwendung als Medikament in der Prävention oder der Behandlung einer Depletion und/oder einer Fehlfunktion der Paneth-Zellen bei einem Patienten,
wobei der Prozentsatz der Identität der Prozentsatz der identischen Aminosäuren zwischen zwei zu vergleichenden Sequenzen ist, wobei die Vergleiche zwischen zwei Polypeptidsequenzen vor der Alignierung dieser zwei Polypeptidsequenzen optimalerweise über ihre Gesamtlänge realisiert werden, wobei die Anzahl der Positionen, für die die Aminosäuren identisch sind, anschließend durch die Gesamtzahl der Aminosäuren der größeren der beiden verglichenen Sequenzen dividiert wird und der Prozentsatz der Identität durch Multiplizieren des erhaltenen Ergebnisses mit 100 erhalten wird.

7. Polypeptid für seine Verwendung gemäß Anspruch 6 als Medikament in der Prävention oder der Behandlung einer Erkrankung ausgewählt aus einer Erkrankung, welche in Verbindung steht mit einem Defekt der Expression und/oder der Sekretion eines Moleküls der Darmabwehr, exprimiert durch Paneth-Zellen, einer Darmentzündung bedingt durch Bakterien, Viren, Hefen oder Protozoen, einer Veränderung der Darmflora, nekrotisierender Enterokolitis des Säuglings, Diarrhö, Morbus Crohn und anderen Darm- und Colonentzündungen.

8. Pharmazeutische Zusammensetzung umfassend ein Polypeptid wie in Anspruch 1 oder 2 definiert als Wirkstoff, zusammen mit einem pharmazeutisch annehmbaren Träger.

9. Verwendung eines isolierten Polypeptids, bestehend aus:
- SEQ ID NO : 1, oder
- einer Sequenz mit mindestens 90 % Identität mit SEQ ID NO : 1, oder
- einer Sequenz aus mindestens 4 aufeinanderfolgenden Aminosäuren umfasst in SEQ ID NO : 1,
welches mindestens einen Effekt aufweist, ausgewählt aus:
- Induktion der Expression und/oder der Sekretion mindestens eines gastrointestinalen Mucins; und
- Induktion der Expression und/oder der Sekretion mindestens eines Moleküls der Darmabwehr, exprimiert durch Paneth-Zellen; und
- Induktion der Erhöhung der Population von Mukuszellen und/oder der Population von Paneth-Zellen,
für die Herstellung einer Nahrungsmittelzusammensetzung,
wobei der Prozentsatz der Identität der Prozentsatz der identischen Aminosäuren zwischen zwei zu vergleichenden Sequenzen ist, wobei die Vergleiche zwischen zwei Polypeptidsequenzen vor der Alignierung dieser zwei Polypeptidsequenzen optimalerweise über ihre Gesamtlänge realisiert werden, wobei die Anzahl der Positionen, für die die Aminosäuren identisch sind, anschließend durch die Gesamtzahl der Aminosäuren der größeren der beiden verglichenen Sequenzen dividiert wird und der Prozentsatz der Identität durch Multiplizieren des erhaltenen Ergebnisses mit 100 erhalten wird.

10. Verwendung gemäß Anspruch 9 zur Herstellung einer milchhaltigen Nahrungsmittelzusammensetzung.

11. Nicht-milchhaltige Nahrungsmittelzusammensetzung umfassend ein Polypeptid bestehend aus:
- SEQ ID NO : 1, oder
- einer Sequenz mit mindestens 90 % Identität mit SEQ ID NO : 1, oder
- einer Sequenz aus mindestens 4 aufeinanderfolgenden Aminosäuren umfasst in SEQ ID NO : 1,
welches mindestens einen Effekt aufweist, ausgewählt aus:
- Induktion der Expression und/oder der Sekretion mindestens eines gastrointestinalen Mucins; und
- Induktion der Expression und/oder der Sekretion mindestens eines Moleküls der Darmabwehr, exprimiert durch Paneth-Zellen; und
- Induktion der Erhöhung der Population von Mukuszellen und/oder der Population von Paneth-Zellen,
wobei das Polypeptid eine Konzentration von zwischen 0,001 und 10µM hat,
wobei der Prozentsatz der Identität der Prozentsatz der identischen Aminosäuren zwischen zwei zu vergleichenden Sequenzen ist, wobei die Vergleiche zwischen zwei Polypeptidsequenzen vor der Alignierung dieser zwei Polypeptidsequenzen optimalerweise über ihre Gesamtlänge realisiert werden, wobei die Anzahl der Positionen, für die die Aminosäuren identisch sind, anschließend durch die Gesamtzahl der Aminosäuren der größeren der beiden verglichenen Sequenzen dividiert wird und der Prozentsatz der Identität durch Multiplizieren des erhaltenen Ergebnisses mit 100 erhalten wird.

12. Milchhaltige Zusammensetzung umfassend ein Polypeptid bestehend aus:
- SEQ ID NO : 1, oder
- einer Sequenz mit mindestens 90 % Identität mit SEQ ID NO : 1, oder
- einer Sequenz aus mindestens 4 aufeinanderfolgenden Aminosäuren umfasst in SEQ ID NO : 1,
welches mindestens einen Effekt aufweist, ausgewählt aus:
- Induktion der Expression und/oder der Sekretion mindestens eines gastrointestinalen Mucins; und
- Induktion der Expression und/oder der Sekretion mindestens eines Moleküls der Darmabwehr, exprimiert durch Paneth-Zellen; und
- Induktion der Erhöhung der Population von Mukuszellen und/oder der Population von Paneth-Zellen,
wobei das Polypeptid eine Konzentration von mindestens 7 µM hat,
wobei der Prozentsatz der Identität der Prozentsatz der identischen Aminosäuren zwischen zwei zu vergleichenden Sequenzen ist, wobei die Vergleiche zwischen zwei Polypeptidsequenzen vor der Alignierung dieser zwei Polypeptidsequenzen optimalerweise über ihre Gesamtlänge realisiert werden, wobei die Anzahl der Positionen, für die die Aminosäuren identisch sind, anschließend durch die Gesamtzahl der Aminosäuren der größeren der beiden verglichenen Sequenzen dividiert wird und der Prozentsatz der Identität durch Multiplizieren des erhaltenen Ergebnisses mit 100 erhalten wird.

13. Nahrungsmittelzusammensetzung gemäß Anspruch 11, wobei das Polypeptid eine Konzentration von 0,01 µM hat.

14. Verfahren zur Herstellung eines durch mindestens einen Stamm von *Lactobacillus delbrueckii* subsp. *bulgaricus* und/oder mindestens einen Stamm von *Streptococcus salivarus* subsp. *thermophilus* fermentierten Milchprodukts, umfassend einen Schritt der Bestimmung der Konzentration eines Polypeptids bestehend aus:
- SEQ ID NO : 1, oder
- einer Sequenz mit mindestens 90 % Identität mit SEQ ID NO : 1, oder
- einer Sequenz aus mindestens 4 aufeinanderfolgenden Aminosäuren umfasst in SEQ ID NO : 1,
welches mindestens einen Effekt aufweist, ausgewählt aus:
- Induktion der Expression und/oder der Sekretion mindestens eines gastrointestinalen Mucins; und
- Induktion der Expression und/oder der Sekretion mindestens eines Moleküls der Darmabwehr, exprimiert durch Paneth-Zellen; und
- Induktion der Erhöhung der Population von Mukuszellen und/oder der Population von Paneth-Zellen,
wobei der Prozentsatz der Identität der Prozentsatz der identischen Aminosäuren zwischen zwei zu vergleichenden Sequenzen ist, wobei die Vergleiche zwischen zwei Polypeptidsequenzen vor der Alignierung dieser zwei Polypeptidsequenzen optimalerweise über ihre Gesamtlänge realisiert werden, wobei die Anzahl der Positionen, für die die Aminosäuren identisch sind, anschließend durch die Gesamtzahl der Aminosäuren der größeren der beiden verglichenen Sequenzen dividiert wird und der Prozentsatz der Identität durch Multiplizieren des erhaltenen Ergebnisses mit 100 erhalten wird.

15. Verfahren gemäß Anspruch 14, wobei der Stamm von *Lactobacillus delbrueckii* subsp. *bulgaricus* und/oder der Stamm von *Streptococcus salivarus* subsp. *Thermophilus* agieren gelassen wird, bis ein vorbestimmter Wert der Konzentration des Polypeptids erreicht ist.

## Claims

1. Isolated polypeptide composed of:
- SEQ ID NO : 1, or
- a sequence having at least 90% identity with SEQ ID NO : 1, or
- a sequence of at least 20 consecutive amino acids contained in SEQ ID NO : 1,
which has at least one effect chosen from:
- induction of the expression and/or secretion of at least one gastrointestinal mucin; and
- induction of the expression and/or secretion of at least one intestinal defence molecule expressed by Paneth cells; and
- induction of the increase in the population of mucous cells and/or in the population of Paneth cells,
the percentage identity being the percentage of identical amino acids between two sequences to be compared, the comparisons between two polypeptide sequences being carried out after the two sequences have been optimally aligned over the totality of their length, the number of positions for which the amino acids are identical then being divided by the total number of amino acids of the largest of the two compared sequences and the percentage identity being obtained by multiplying the result obtained by 100.

2. Polypeptide according to claim 1, composed of SEQ ID NO : 1.

3. Polypeptide according to claim 1 or 2, for use as a medicament.

4. Polypeptide composed of:
- SEQ ID NO : 1, or
- a sequence having at least 90% identity with SEQ ID NO : 1, or
- a sequence of at least four consecutive amino acids contained in SEQ ID NO : 1,
which has at least one effect chosen from:
- induction of the expression and/or secretion of at least one gastrointestinal mucin; and
- induction of the expression and/or secretion of at least one intestinal defence molecule expressed by Paneth cells; and
- induction of the increase in the population of mucous cells and/or in the population of Paneth cells,
for use as a medicament in the treatment of a defect of expression and/or secretion of gastrointestinal mucins and/or of an alteration of the gastrointestinal mucosal barrier in a patient,
the percentage identity being the percentage of identical amino acids between two sequences to be compared, the comparisons between two polypeptide sequences being carried out after the two sequences have been optimally aligned over the totality of their length, the number of positions for which the amino acids are identical then being divided by the total number of amino acids of the largest of the two compared sequences and the percentage identity being obtained by multiplying the result obtained by 100.

5. Polypeptide for use according to claim 4 as a medicament in the prevention or treatment of a pathology selected from the group consisting of intestinal inflammation, especially chronic intestinal inflammation, an intestinal infection, gastric or duodenal ulcers especially due to a *Helicobacter pylori* infection, diarrhoea, constipation, intestinal and colon cancers, and mucositis induced by radiotherapy or chemotherapy.

6. Polypeptide composed of:
- SEQ ID NO : 1, or
- a sequence having at least 90% identity with SEQ ID NO : 1, or
- a sequence of at least four consecutive amino acids contained in SEQ ID NO : 1,
which has at least one effect chosen from:
- induction of the expression and/or secretion of at least one gastrointestinal mucin; and
- induction of the expression and/or secretion of at least one intestinal defence molecule expressed by Paneth cells; and
- induction of the increase in the population of mucous cells and/or in the population of Paneth cells,
for use as a medicament in the prevention or treatment of Paneth cell depletion and/or dysfunction in a patient,
the percentage identity being the percentage of identical amino acids between two sequences to be compared, the comparisons between two polypeptide sequences being carried out after the two sequences have been optimally aligned over the totality of their length, the number of positions for which the amino acids are identical then being divided by the total number of amino acids of the largest of the two compared sequences and the percentage identity being obtained by multiplying the result obtained by 100.

7. Polypeptide for use according to claim 6, as a medicament in the prevention or treatment of a pathology chosen from a pathology associated with a defect of expression and/or secretion of an intestinal defence molecule expressed by Paneth cells, an intestinal infection due to bacteria, viruses, yeasts or protozoa, an alteration in the intestinal microbiota, necrotising enterocolitis in infants, diarrhoea, Crohn's disease and other intestinal and colonic inflammations.

8. Pharmaceutical composition comprising a polypeptide as defined in claim 1 or 2 as active ingredient, in association with a pharmaceutically acceptable carrier.

9. Use of an isolated polypeptide composed of:
- SEQ ID NO : 1, or
- a sequence having at least 90% identity with SEQ ID NO : 1, or
- a sequence of at least four consecutive amino acids contained in SEQ ID NO : 1,
which has at least one effect chosen from:
- induction of the expression and/or secretion of at least one gastrointestinal mucin; and
- induction of the expression and/or secretion of at least one intestinal defence molecule expressed by Paneth cells; and
- induction of the increase in the population of mucous cells and/or in the population of Paneth cells,
in the preparation of a food composition,
the percentage identity being the percentage of identical amino acids between two sequences to be compared, the comparisons between two polypeptide sequences being carried out after the two sequences have been optimally aligned over the totality of their length, the number of positions for which the amino acids are identical then being divided by the total number of amino acids of the largest of the two compared sequences and the percentage identity being obtained by multiplying the result obtained by 100.

10. Use according to claim 9 in the preparation of a dairy food composition.

11. Non-dairy food composition comprising a polypeptide composed of:
- SEQ ID NO : 1, or
- a sequence having at least 90% identity with SEQ ID NO : 1, or
- a sequence of at least four consecutive amino acids contained in SEQ ID NO : 1,
which has at least one effect chosen from:
- induction of the expression and/or secretion of at least one gastrointestinal mucin; and
- induction of the expression and/or secretion of at least one intestinal defence molecule expressed by Paneth cells; and
- induction of the increase in the population of mucous cells and/or in the population of Paneth cells,
wherein the polypeptide is in a concentration between 0.001 and 10 µM,
the percentage identity being the percentage of identical amino acids between two sequences to be compared, the comparisons between two polypeptide sequences being carried out after the two sequences have been optimally aligned over the totality of their length, the number of positions for which the amino acids are identical then being divided by the total number of amino acids of the largest of the two compared sequences and the percentage identity being obtained by multiplying the result obtained by 100.

12. Dairy food composition comprising a polypeptide composed of:
- SEQ ID NO : 1, or
- a sequence having at least 90% identity with SEQ ID NO : 1, or
- a sequence of at least four consecutive amino acids contained in SEQ ID NO : 1,
which has at least one effect chosen from:
- induction of the expression and/or secretion of at least one gastrointestinal mucin; and
- induction of the expression and/or secretion of at least one intestinal defence molecule expressed by Paneth cells; and
- induction of the increase in the population of mucous cells and/or in the population of Paneth cells,
wherein the polypeptide is in a concentration of at least 7 µM,
the percentage identity being the percentage of identical amino acids between two sequences to be compared, the comparisons between two polypeptide sequences being carried out after the two sequences have been optimally aligned over the totality of their length, the number of positions for which the amino acids are identical then being divided by the total number of amino acids of the largest of the two compared sequences and the percentage identity being obtained by multiplying the result obtained by 100.

13. Food composition according to claim 11, wherein the polypeptide is in a concentration equal to 0.01 µM.

14. Process for the preparation of a dairy product fermented by at least one strain of *Lactobacillus delbrueckii* ssp. *bulgaricus* and/or at least one strain of *Streptococcus salivarus* ssp. *thermophilus,* comprising a step of determining the concentration of a polypeptide composed of:
- SEQ ID NO : 1, or
- a sequence having at least 90% identity with SEQ ID NO : 1, or
- a sequence of at least four consecutive amino acids contained in SEQ ID NO : 1,
which has at least one effect chosen from:
- induction of the expression and/or secretion of at least one gastrointestinal mucin; and
- induction of the expression and/or secretion of at least one intestinal defence molecule expressed by Paneth cells; and
- induction of the increase in the population of mucous cells and/or in the population of Paneth cells,
the percentage identity being the percentage of identical amino acids between two sequences to be compared, the comparisons between two polypeptide sequences being carried out after the two sequences have been optimally aligned over the totality of their length, the number of positions for which the amino acids are identical then being divided by the total number of amino acids of the largest of the two compared sequences and the percentage identity being obtained by multiplying the result obtained by 100.

15. Process according to claim 14, wherein the strain of *Lactobacillus delbrueckii* ssp. *bulgaricus* and/or the strain of *Streptococcus salivarus* ssp. *thermophilus* is allowed to act until a predetermined value of the concentration of the polypeptide is reached.
